# EUROPÄISCHE PATENTSCHRIFT

(11) **EP 1 049 465 B1**
(45) Veröffentlichungstag und Bekanntmachung des Hinweises auf die Patenterteilung: **11.10.2006**
(21) Anmeldenummer: 99903669.2
(22) Anmeldetag: 22.01.1999
(51) Int. Cl.: A61K 31/40, C07K 14/71, C07K 16/28, C12N 15/12, C12Q 1/68, A61P 35/00

(54) **VERWENDUNG VON INHIBITOREN FÜR DIE BEHANDLUNG VON RTK-ÜBERFUNKTIONS-BEDINGTEN STÖRUNGEN, INSBESONDERE VON KREBS**
USE OF INHIBITORS FOR THE TREATMENT OF DISORDERS RELATED TO RTK HYPERFUNCTION, ESPECIALLY CANCER
UTILISATION D'INHIBITEURS POUR LE TRAITEMENT DE TROUBLES LIES A UNE HYPERFONCTION DE RECEPTEURS PRESENTANT UNE ACTIVITE DE TYROSINE KINASE (RTK), NOTAMMENT LE CANCER

(30) Priorität: 22.01.1998 DE 19802377
(43) Veröffentlichungstag der Anmeldung: 08.11.2000
(73) Patentinhaber: Max-Planck-Gesellschaft zur Förderung der Wissenschaften e.V., 80539 München (DE)
(72) Erfinder: ULLRICH, Axel, D-80799 München (DE); BANGE, Johannes, D-81373 München (DE); KNYAZEV, Pjotr, D-82131 Gauting (DE)
(74) Vertreter: Grünecker, Kinkeldey, Stockmair & Schwanhäusser Anwaltssozietät
(86) Internationale Anmeldenummer: PCT/EP1999/000405
(87) Internationale Veröffentlichungsnummer: WO 1999/037299

(56) Entgegenhaltungen:
- WO-A-97/13771
- WO-A-98/24432
- WO-A-98/50356
- MOHAMMADI M ET AL: "STRUCTURES OF THE TYROSINE KINASE DOMAIN OF FIBROBLAST GROWTH FACTOR RECEPTOR IN COMPLEX WITH INHIBITORS" SCIENCE, Bd. 276, Nr. 5314, 9. Mai 1997, Seiten 955-960, XP002065235 in der Anmeldung erwähnt
- PANEK R.L.: 'In Vitro Pharmacological Characterization of PD 166285' JPET Bd. 283, Nr. 3, Seiten 1433 - 1444
- BATLEY B.L.: 'Inhibition of FGF-1 Receptor Tyrosine Kinase Activity...' LIFE SCIENCES Bd. 62, Nr. 2, 1998, Seiten 143 - 150
- HAMBY J.M.: 'Structure-Activity Relationships for...' J MED CHEM Bd. 40, 1997, Seiten 2296 - 2303
- HUDZIAK R.M.: 'Monoclonal Antibody Has Antiproliferative Effecs...' MOLECULAR AND CELLULAR BIOLOGY Bd. 9, Nr. 3, 1989, Seiten 1165 - 1172
- HOLLIS SHOWALTER H.D.: 'Small Molecule Inhibitors...' PHARMACOL THER Bd. 76, Nr. 1-3, 1997, Seiten 55 - 71
- CLARK R.: 'Antibosies specific for amino acid 12 of...' PROC. NATL. ACAD. SCI. Bd. 82, 1985, Seiten 5280 - 5284
- DINNEY C.P. ET AL: 'Therapy of human transitional cell carcinoma of the bladder..' CLINICAL CANCER RESEARCH Bd. 3, Nr. 2, 1997, Seiten 161 - 168
- PARTANEN J. ET AL: 'FGFR-4, a novel acidic fibroblast growth factor receptor...' EMBO J Bd. 10, Nr. 6, 1991, Seiten 1347 - 1354
- NORMANNO N. ET AL: 'Growth Inhibition of human colon carcinoma cells by...' CLINICAL CANCER RESEARCH Bd. 2, Nr. 3, 1996, Seiten 601 - 609
- ABRAMS S.I.: 'Mutant ras epitopes as targets for cancer vaccines' SEMIN ONCOL. Bd. 23, Nr. 1, 1996, Seiten 118 - 134
- UENO H.: 'A Truncated Form of Fibroblast Growth Factor Receptor 1 inhibits...' THE JOURNAL OF BIOLOGICAL CHEMISTRY Bd. 267, Nr. 3, 1992, Seiten 1470 - 1476
- YAN LI ET AL: 'Cell Transformation by Fibroblast' MOLECULAR AND CELLULAR BIOLOGY Bd. 14, Nr. 11, 1994, Seiten 7660 - 7669

## Beschreibung

Die vorliegende Erfindung betrifft die Verwendung von Inhibitoren für die Behandlung und/oder Prophylaxe von Krankheiten, die Folge einer erhöhten Rezeptortyrosinkinaseaktivität sind, insbesondere von Krebs. Die Verwendung ist insbesondere auf eine Inhibition oder Verminderung der Überexpression und/oder veränderten Aktivität von Rezeptortyrosinkinasen (RTKs) gerichtet. Insbesondere kann diese veränderte Aktivität der Rezeptortyrosinkinase durch eine Mutation des FGFR-4 ausgelöst werden, wobei diese Mutation insbesondere eine Punktmutation in der Transmembrandomäne von FGFR-4 ist und zu einem Austausch einer hydrophoben gegen eine hydrophile Aminosäure führt. Die Erfindung betrifft ferner die Verwendung der Inhibitoren der FGFR-Kinasen, insbesondere für die Behandlung und/oder Prophylaxe von Krebs. Weiterhin betrifft die Erfindung einen mutierten FGFR-4, der zu einer Überexpression und/ oder veränderten Aktivität in Zellen führt. Die Erfindung betrifft schließlich eine DNA- und RNA-Sequenz eines mutierten FGFR-4-Moleküls. Schließlich betrifft die Erfindung außerdem eine pharmazeutische Zusammensetzung, umfassend den Inhibitor, wie oben beschrieben, ferner ein Diagnose- und Screeningverfahren.

Zellwachstum ist ein sorgfältig regulierter Prozeß in Abhängigkeit von den speziellen Bedürfnissen eines Organismus. Bei einem jungen Organismus überwiegt die Zellteilungsrate die Absterberate von Zellen, was zu einer Größenzunahme des Organismus führt. Bei einem ausgewachsenen Organismus sind die Neubildungen von Zellen und der Zelltod so ausgewogen, daß ein "Fließgleichgewicht" entsteht. In seltenen Fällen bricht jedoch die Kontrolle der Zellvermehrung zusammen und die Zellen beginnen zu wachsen und sich zu teilen, obwohl in dem Organismus kein spezieller Bedarf für eine höhere Anzahl von Zellen dieses Typs besteht. Dieses unkontrollierte Zellwachstum ist die Ursache von Krebs. Faktoren, die das unkontrollierte Zellwachstum, teilweise verbunden mit Metastasenbildung, hervorrufen können, sind oftmals chemischer Natur, können jedoch auch physikalischer Art, wie z.B. radioaktive Strahlung, sein. Eine andere Ursache für die Auslösung von Krebs sind genetische Eigenheiten oder Mutationen in einem bestimmten Organismus, die früher oder später dazu führen, daß Zellen entarten.

Die Verfahren, die das normale Wachstum und die Differenzierung z.B. in der Brust steuern, konnten bis jetzt noch nicht in zufriedenstellender Weise aufgeklärt werden. Zusätzlich zu einer hormonellen Steuerung kommt ein komplexes Netzwerk verschiedener, lokal erzeugter Wachstumsfaktoren hinzu, die in die Entwicklung der Brustdrüsenzellen eingreifen. Die genauen Ursachen für die Entstehung von Krebs bei Brustdrüsenzellen ist genauso unklar und unbekannt wie auch vielfältig, entsprechend der Situation bei anderen Zellen. Veränderungen von Onkogenen und Tumorsupressorgenen scheinen eine wichtige Rolle bei der Karzinogenese des Brustkrebs zu spielen. Zusätzlich kann eine verstärkte Stimulation durch regulatorische Faktoren, die in genetisch veränderten Zellen auftreten, zu einem vermehrten Fortschreiten des Zellwachstums führen.

Zur Therapie von Krebs stehen gegenwärtig im wesentlichen zwei Alternativen zur Verfügung. Entweder gelingt es, die Krebszellen durch einen operativen Eingriff vollständig aus dem erkrankten Organismus zu entfernen, oder es wird versucht, die entarteten Zellen im Organismus unschädlich zu machen, wie beispielsweise durch Verabreichung von Medikamenten (Chemotherapie) oder durch physikalische Therapieverfahren, wie Bestrahlung.

Bei der Chemotherapie werden häufig Medikamente eingesetzt, die in irgendeiner Form in den DNA-Stoffwechsel eingreifen und schnell wachsende Zellen, die eine höhere DNA-Stoffwechselleistung erbringen müssen, stärker schädigen als Zellen, die sich nicht oder nur langsam teilen. Ein schwerwiegender Nachteil vieler Chemotherapeutika ist jedoch die geringe Spezifität der verwendeten Wirkstoffe, was zur Folge hat, daß auch gesunde Zellen bei der Chemotherapie geschädigt werden. Diese geringe Spezifität der Wirkstoffe fordert weiterhin, daß deren Dosierung jeweils so erfolgen muß, das möglichst wenig gesunde Zellen bei gleichzeitiger Abtötung der Krebszellen geschädigt werden. Dies ist oftmals nicht möglich und der krebskranke Patient stirbt aufgrund der sich immer weiter ausbreitenden Krebszellen, die im Endstadion den Ausfall lebenswichtiger Funktionen herbeiführen.

Es wird angenommen, daß die Überexpression und/oder veränderte Aktivität bestimmter Wachstumsfaktorrezeptoren zu dem verstärkten Wachstum vieler Neoplasmen, einschließlich bei Brustkrebs, beitragen. Zum Beispiel wurde die Überexpression von EGFR, d. h. dem Epidermal Factor Receptor, oder dem ERB B-2-Rezeptor in Brusttumoren mit einer schlechten Prognose in Verbindung gebracht. Auch FGF- (historisch: Fibroblast Growth Factor) Proteine könnten eine Rolle bei der Entwicklung von Krebs in Brustdrüsen oder von anderem Krebs spielen; die Ergebnisse in dieser Richtung widersprechen sich jedoch oder sind nicht konklusiv.

Die FGFs bilden eine große Familie von peptidregulatorischen Faktoren, von denen bis jetzt 9 Mitglieder bekannt sind. Acht davon sind in Menschen gut charakterisiert worden (Basilico und Moscattelli, 1992; Coulier et al., 1993). Die FGFs wirken über Hochaffinitäts-Tyrosinkinaserezeptoren, die durch mindestens vier unterschiedliche Gene kodiert werden. Die FGFs sind weiterhin multifunktionelle, regulatorische Peptide, die nicht nur auf die Tumorgenese wirken könnten, sondern auch bei kardiovaskulären Krankheiten, dem Aufbau nach einer Gewebsverletzung, der Neurobiologie und der Embryonalentwicklung eine große Rolle spielen könnten. Die sauren und basischen FGFs (aFGF und bFGF) waren die ersten und sind die am besten gekennzeichneten Familienmitglieder. In vivo konnte für FGFs z. B. nachgewiesen werden, daß sie an einer mesodermalen Induktion in der Embryogenese teilnehmen (Slack et al., 1987; Kimelman et al., 1988), wie auch eine Beteiligung an der Angiogenese (Thomas et al., 1985; Thompson et al., 1989; Folkmann and Klagsbrun, 1987).

Für die korrespondierenden Rezeptoren (FGFRs) wurden vier ähnliche Gene identifiziert, die für sie kodieren. Diese Gene kodieren strukturell verwandte Proteine mit einer extrazellulären Domäne, die aus drei Immunglobulinloops besteht und einem sauren Anteil, einer hydrophoben Transmembrandomäne sowie einer intrazellulären Domäne, welche eine Tyrosinkinaseaktivität einschließt. Für zwei dieser Gene, FGFR-1 und FGFR-2, konnte gezeigt werden, daß sie multiple Transkripte haben, die über alternatives Spleißen entstehen (Givol und Yayon, 1992 und Johnson und Williams 1993). Spleiß-Varianten, die sich aus diesen Genen ergeben, unterscheiden sich im Hinblick auf die Anzahl der Immunglobulin-ähnlichen Domänen im extrazellulären Bereich des Rezeptors und in der Sequenz für die zweite Hälfte der dritten Immunglobulindomäne, die aus alternativen Exons entstehen kann. Außerdem können Transmembran- und Juxtamembran-Verkürzungen oder Deletionen auftreten, die sekretierte oder Kinase-inaktive Proteinprodukte erzeugen können.

Für FGFR-3 konnten alternative Transkripte und entsprechende Isoformen gefunden werden, aber für FGFR-4 gibt es nur ein einziges bekanntes Proteinprodukt. Aufgrund der

Vielzahl der FGFR-Gene und Transkripte und des Mangels vieler Proteinprodukte an einer Spezifität für bestimmte FGFs ist es schwierig, die Wirkung eines spezifischen Liganden auf einen spezifischen Rezeptor zu bestimmen. Korrelationen zwischen spezifischen FGF-Rezeptoren und bestimmten Krankheiten sind daher nur sehr schwierig zu etablieren, geschweige denn eine Korrelation eines bestimmten Wirkungsmechanismusses eines bestimmten Rezeptors mit einer Krankheit. Dementsprechend schwierig ist es, Krankheiten, insbesondere das komplexe Krankheitsbild Krebs, wirksam unter Ausnutzung der FGFRs zu therapieren.

Es wurden ebenfalls Inhibitoren von Rezeptortyrosinkinasen gefunden. Mohammadi et al. beschreiben besipielsweise Strukturen der Tyrosin Kinase Domäne des Wachstumsfaktor-Rezeptors aus Fibroblasten FGFR-1 im Komplex mit inhibitoren. Dabei wird eine Reihe von synthetischen Verbindungen auf der Basis von Indolon-Strukturen auf ihre Wirksamkeit als Inhibitor gescreent. Mohammadi et al beschreiben damit eine neue Klasse an Protein-Tyrosin-Kinase-Inhibitoren, die durch Kopplung verschiedener chemischer Substituenten an den Oxindol Ken (Indolinone) hergestellt wurden. Mohammadi et al. beschreiben keine Inhibitoren von FGFR-4 oder mutiertem FGFR-4.

WO 97/13771 beschreibt bizyklische heteroaromatische Verbindungen, die als Inhibitoren der Rezeptortyrosinkinasen c-erB-2, EGF-R und PDGF-R geeignet sind. Weiterhin beschreibt WO 97/13771 ein Verfahren zu deren Herstellung, pharmazeutische Verbindungen enthaltend diese Inhibitoren und die Behandlung von Krebs. WO 97/13771 beschreibt keine Inhibitoren von FGFR-4 oder mutiertem FGFR-4.

Es ist daher eine Aufgabe der vorliegenden Erfindung, eine Möglichkeit zur Behandlung und/oder Prophylaxe von körperlichen Beeinträchtigungen anzugeben, an deren Entstehung Rezeptortyrosinkinasen (RTKs) beteiligt sind, insbesondere von Krebs. Insbesondere ist es eine Aufgabe der vorliegenden Erfindung, eine Überexpression und/oder veränderte, beispielsweise konstitutive Aktivität von Rezeptortyrosinkinasen zu hemmen und/oder zu erniedrigen.

Es ist ferner eine Aufgabe der vorliegenden Erfindung, die veränderte Aktivität der Rezeptortyrosinkinase eines mutierten FGFR-4 zu hemmen oder zu erniedrigen.

Es ist eine weitere Aufgabe der vorliegenden Erfindung, eine weitere RTK anzugeben, die an der Krebsentstehung und/oder Metastasierung beteiligt ist. Femer ist es eine Aufgabe der vorliegenden Erfindung, eine DNA-Sequenz oder korrespondierende RNA-Sequenz der RTK anzugeben.

Es ist eine weitere Aufgabe der vorliegenden Erfindung, verbesserte Diagnose- oder Differentialdiagnose- und Screeningverfahren anzugeben.

Schließlich ist es eine Aufgabe der vorliegenden Erfindung, eine pharmazeutische Zusammensetzung anzugeben, mit der insbesondere Krebs behandelt werden kann.

Diese Aufgaben werden durch die Gegenstände der unabhängigen Ansprüche gelöst. Die abhängigen Ansprüche geben bevorzugte Weiterbildungen der Erfindung an. Zum besseren Verständnis der vorliegenden Erfindung werden im folgenden die hierin verwendeten Begriffe näher erläutert.

Unter "Inhibitor" wird jede Substanz verstanden, die die RTK hemmt oder ihre Aktivität erniedrigt. Dabei kann es sich um eine gegen die RTK gerichtete niedermolekulare Substanz, einen Kinase-inaktiven Rezeptor oder einen Anti-Rezeptor-Antikörper handeln.

Unter einem "Kinase-inaktiven Rezeptor" wird ein Rezeptor verstanden, der keine Tyrosinkinaseaktivität mehr besitzt.

Unter "Rezeptortyrosinkinase" wird jeder Rezeptor verstanden, der Tyrosinkinaseaktivität besitzt. Der Ausdruck umfaßt Wachstumsfaktorrezeptoren, die Tyrosinkinaseaktivität besitzen, wie auch HER2 oder die met-Rezeptoren.

Unter "RTK-Überfunktion" wird eine Überexpression (siehe unten) und/oder veränderte Aktivität (siehe unten) verstanden.

"Defekte Signalübertragsungsaktivitäten" bedeutet, daß ein mutierter Rezeptor nicht mehr in der Lage ist, ein extrazelluläres Wachstumssignal oder ein anderes Signal in der Weise in ein intrazelluläres Signal umzusetzen, wobei diese defekte Signalerzeugung nicht mehr von der Anwesenheit eines Liganden, beispielsweise des Wachstumsfaktors, abhängt.

"Wachstumsfaktor" bedeutet jede mitogene Chemikalie, überlicherweise ein Polypeptid, das u.a. von normalen und/oder transformierten Säugerzellen ausgeschieden wird, und das eine wesentliche Rolle bei der Regulation des Zellwachstums spielt, insbesondere bei der Stimulierung der Proliferation der Zellen und dem Aufrechterhalten ihrer Lebensfähigkeit. Der Begriff "Wachstumsfaktor" umfaßt z.B. den epidermalen Wachstumsfaktor (EGF), den von Plättchen stammenden Wachstumsfaktor (PDGF) und den Nervenwachstumsfaktor (NGF), wie auch den FGF, also den Fibroblastenwachstumsfaktor.

Unter "mutierter Rezeptortyrosinkinase" wird eine Rezeptortyrosinkinase verstanden, die im Vergleich zu dem Wildtyp-Rezeptor eine Strukturänderung enthält, so daß der Rezeptor eine andere, z.B. nicht mehr regulierbare Tyrosinkinaseaktivität als der Wildtyp-Rezeptor besitzt. Eine Klasse von Mutationen führt zu einer veränderten Aktivität der RTK.

Unter "Wildtyp-Wachstumsfaktorrezeptor" bzw. "Wildtyp"-Rezeptor wird ein natürlich vorkommender Wachstumsfaktorrezeptor oder Rezeptor verstanden, der die nicht mutierte Aminosäuresequenz trägt. Der "Wildtyp" entspricht der in der Population am häufigsten vorkommenden Rezeptorvariante.

Unter "extrazellulärer Domäne" des Wachstumsfaktorrezeptors oder Rezeptors wird der Teil des Rezeptors verstanden, der normalerweise aus der Zelle in die extrazelluläre Umgebung herausragt. Die extrazelluläre Domäne umfaßt beispielsweise den Teil des Rezeptors, an dem ein Wachstumsfaktor oder ein anderes Molekül (Ligand) bindet.

Unter "Transmembranregion" des Wachstumsfaktorrezeptors oder Rezeptors wird der hydrophobe Anteil des Rezeptors verstanden, der normalerweise in der Zellmembran der Zelle lokalisiert ist, die den Rezeptor exprimiert.

Unter "Tyrosinkinasedomäne" oder "zytoplasmatischer Domäne" des Wachstumsfaktorrezeptors oder Rezeptors wird der Teil des Rezeptors verstanden, der sich normalerweise innerhalb der Zelle befindet, und der die Transphosphorylierung von Tyrosinresten bewirkt.

Unter "einer wirksamen Menge" wird eine Menge der erfindungsgemäßen Zusammensetzung verstanden, die den gewünschten therapeutischen Effekt erzielen kann.

Unter "Fibroblasten-Wachstumsfaktor (FGF)" wird ein mitogenes Polypeptid verstanden, daß das Wachstum und andere Eigenschaften von Zellen, u. a. von Fibroblasten, beeinflußt.

Unter "Überexpression" wird eine vermehrte Herstellung von RTK-Protein durch eine Zelle im Vergleich zum Wildtyp verstanden. Dies kann z.B. durch Genamplifikation des RTK-Gens ausgelöst werden und zu einer überschüssigen, unkontrollierten Zellteilungsaktivität führen.

Unter "veränderter Aktivität" wird eine ständige Aktivität eines durch Wachstumsfaktorrezeptoren vermittelten Signalübertragungswegs verstanden. So ist bei einer veränderten RTK die Kinaseaktivität auch dann vorhanden, wenn kein Ligand vorliegt.

Gemäß der vorliegenden Erfindung konnte gezeigt werden, daß ein mutierter FGFR-4 zu einer Überexpression und/oder veränderter Aktivität der entsprechenden Rezeptortyrosinkinase in Zellen und damit zu Krebs führen kann.

Wachstumsfaktorrezeptoren spielen bei der Entstehung und Vermehrung von menschlichen Krebszellen eine entscheidende Rolle. Bei gesunden Zellen sind die Wachstumsfaktorrezeptoren u.a. an der Kontrolle des Zellwachstums, aber auch an der Differenzierung, Zellmigration etc. beteiligt. Das eigentliche Signal zur Zellteilung ist der Wachstumsfaktor, der in Abhängigkeit von den Bedürfnissen des Organismus gebildet wird. Der Rezeptor übernimmt die Funktion der Signalübertragung, d. h. er ist an der Umsetzung des extrazellulären Wachstumssignals in Zellteilungsaktivität im Inneren der Zelle beteiligt. Bei vielen Wachstumsfaktorrezeptoren spielt deren Fähigkeit, nach Bindung des Wachstumsfaktors an die extrazelluläre Domäne Phosphatreste an Tyrosinreste in Proteinen zu übertragen, eine entscheidende Rolle. Diese Rezeptoren werden auch als Rezeptortyrosinkinasen bezeichnet. Eine Übersicht über Rezeptortyrosinkinasen befindet sich in Yarden, Y. und Ullrich A., Rev. Biochem. 1988, 57, 443 - 78. Die Dimerisierung dieser Wachstumsfaktorrezeptoren nach Bindung des Wachstumsfaktors ist ein weiterer wichtiger Vorgang bei dem Prozeß der Signalübertragung. Die Umwandlung eines extrazellulären Signals in ein intrazelluläres Signal unter Vermittlung von Wachstumsfaktorrezeptoren mit Tyrosinkinaseaktivität läßt sich in die folgenden fünf Stufen zerlegen:
1. Die Bindung des Wachstumsfaktors (auch als Ligand bezeichnet) an die extrazelluläre Domäne des Rezeptors induziert eine Konformationsänderung; diese bewirkt
2. Dimerisierung von Rezeptoren mit veränderter Konformation; mit
3. gleichzeitiger Induktion einer Kinaseaktivität;
4. Transphosphorylierung von Tyrosinresten in dem Rezeptordimer, die wiederum eine aktivierte Rezeptorkonformation erzeugt und stabilisiert; und
5. Phosphorylierung von Polypeptidsubstraten und Wechselwirkung mit zellulären Faktoren.

Eine unkontrollierte Überfunktion dieser Signalübertragungskette beispielsweise aufgrund der Überexpression oder eine veränderte Aktivität des Rezeptors kann unter anderem zu einer überhöhten Teilungsaktivität der entsprechenden Zelle führen und im Extremfall zu einer entarteten Krebszelle. Eine Übersicht über Wachstumsfaktorrezeptoren und deren Funktion bei der Signalübertragung von dem extrazellulären in das intrazelluläre Milieu, sowie der mögliche Einfluß von abnormal exprimierten Rezeptoren auf die Krebsentstehung ist in Ullrich, A. und Schlessinger, J. (1990) Cell 61, 203 - 212 gegeben.

Es wurde nun überraschend gefunden, daß es in der oben erläuterten, fünfstufigen Signalübertragungskette durch einen mutierten FGFR-4 zu einer gesteigerten Signalübertragungsaktivität kommt, an deren Entstehung die veränderte Aktivität mutierter RTK maßgeblich beteiligt ist.

Gemäß Anspruch 1 der vorliegenden Erfindung wird daher mindestens ein Inhibitor einer Rezeptortyrosinkinase für die Behandlung und/oder Prophylaxe von RTK-Überfunktionsbedingten Beeinträchtigungen, insbesondere von Krebs, verwendet. Darüber hinaus können erfindungsgemäß auch Krankheiten bzw. körperliche Beeinträchtigungen beseitigt bzw. gelindert werden, die Folge einer auf eine gesteigerte Signalübertragung zurückzuführenden Hyperproliferation von Gewebe und/oder gesteigerten Invasivität von Gewebe sind.

Als Inhibitor kann neben niedermolekularen Substanzen beispielsweise mindestens ein Kinase-inaktiver Rezeptor verwendet werden. Durch die Verwendung des Inhibitors, z.B. des Kinase-inaktiven Rezeptors kann die veränderte Aktivität der Rezeptortyrosinkinase gehemmt und/oder erniedrigt werden. Wie bereits früher ausgeführt ist die Überexpression und/oder veränderte Aktivität von Wachstumsfaktorrezeptoren ein wichtiger Faktor bei der Auslösung oder dem Fortschreiten von Krebs. Die Überexpression von EGFR oder des Erb B-2-Rezeptors in Brusttumoren wurde z. B. mit einer schlechten Prognose assoziiert (siehe oben). Daher ist eine Hemmung dieser Überexpression und/oder veränderten Aktivität ein wichtiger Bestandteil bei der Behandlung und/oder Prophylaxe von Krebs. FGFR-4 wird während der Embryogenese gewebespezifisch abgeschaltet. Dennoch findet er sich bei 30% der Brustkrebspatientinnen; im Gewebe von Gesunden ist er nicht nachweisbar. Der Einsatz von Inhibitoren für Rezeptortyrosinkinasen führt zu einer Erniedrigung oder vollständigen Inhibition der Überexpression und/oder veränderten Aktivität. Ebenso führt der Einsatz Kinase-inaktiver Rezeptoren zu einer Erniedrigung und/oder vollständigen Inhibition der Aktivität der Rezeptortyrosinkinasen, da die Kinasefunktion des Heterodimers nicht mehr zu einer Signalübertragung fähig ist. Die Wirkung Kinase-inaktiver Rezeptoren beruht darauf, daß nicht funktionelle Heterodimere gebildet werden (Verdünnungseffekt). Eine fehlende Signalübertragung führt zu einer Verhinderung der Weiterleitung des überexprimierten und/oder veränderten aktiven Signals, wodurch verhindert wird, daß das Signal in eine biologische Antwort der Zelle umgesetzt wird. Dadurch kann durch diese Inhibition der Rezeptortyrosinkinase oder durch diese Kinase-inaktiven Rezeptoren wirksam und in positiver Weise in die Behandlung und/oder Prophylaxe von Krebs eingegriffen werden.

Überraschenderweise wurde gefunden, daß die FGFR-4-Mutation auch in der Keimbahn von gesunden Menschen vorkommt. Es wird davon ausgegangen, daß die Keimbahnmutation zu einer genetischen Prädisposition führt, die die betroffenen Personen anfällig macht gegenüber dem Ausbruch verschiedener Erkrankungen. Im Zusammenhang mit der Krebsentstehung wird angenommen, daß die erhöhte Expression des mutierten Rezeptors im Tumorgewebe an der Kanzerogenese beteiligt ist. Die Keimbahnmutation wird weiterhin als Prädisposition u.a. für folgende Erkrankungen angesehen: Arteriosklerose, Leukämie, Lymphome, Leberzellcarcinome sowie Cholangiocarcinome.

Demzufolge stellt die vorliegende Erfindung einen weiteren genetischen Marker zur Verfügung, der sich als äußerst hilfreich in der Diagnose und Früherkennung verschiedener Erkrankungen und Anfälligkeit gegenüber diesen erweist.

Die vorliegende Erfindung betrifft daher auch ein Verfahren zum Nachweis einer Nukleinsäure, die FGFR-4 kodiert, in Patientenmaterial, wobei insbesondere Mutationen der Rezeptor-kodierenden Nukleinsäure nachgewiesen werden. Dies kann beispielsweise durch Hybridisierung mit Oligonukleotidproben erfolgen, die spezifisch die Anwesenheit bzw. Abwesenheit einer Mutation, insbesondere einer Punktmutation, anzeigen können. Dabei wird beispielsweise ein "mismatch" zwischen mutierter Nukleinsäure und Oligonukleotidprobe so ausgenutzt, daß bei vorliegendem "mismatch" eine Hybridisierung nicht erfolgt und somit ein Signal ausbleibt. Alternativ können Mutationen auch durch Amplifikation der Nukleinsäure mit spezifischen FGFR-4-PCR-Primern und anschließender Spaltung mit geeigneten Restriktionsendonukleasen nachgewiesen werden. Betrifft beispielsweise eine Mutation die Erkennungssequenz einer Restriktionsendonuklease, indem beispielsweise die mutierte Erkennungssequenz von der Restriktionsnuklease nicht mehr als Spaltstelle erkannt wird, führt dies zu einem anderen Restriktionsfragment als im nicht-mutierten Wildtyp. Mit Hilfe der PCR lassen sich gezielt Restriktionsfragmente nachweisen, so daß in dem genannten Fall beispielsweise ein größeres Restriktionsfragment in der Mutante vorliegt im Vergleich zum Wildtyp. Alternativ kann jedoch auch eine Mutation zur Entstehung einer neuen Restriktionsspaltstelle führen, wobei sich ein "Wildtyp-Fragment" nach Spaltung mit dem entsprechenden Enzym in der Mutante verkleinert. Die Mutation in der Transmembrandomäne des FGFR-4, an Position 388 der Sequenz, wie in der EMBL GenBank /DDBJ unter X57205 hinterlegt , die zu einem Austausch von Gly im Wildtyp gegen Arg in der Mutante führt, betrifft die Erkennungssequenz GGWCC der Restriktionsendonuklease BstNI. Dadurch entstehen zwei neue Restriktionsfragmente von 80 und 29 bp, die u.a. durch Restriktionsanalyse nachgewiesen werden können.

Gemäß der vorliegenden Erfindung konnte ferner gezeigt werden, daß eine Überexpression und insbesondere eine veränderte Aktivität der RTK zu einer vermehrten Invasivität, d.h. einer verstärkten Metastasenbildung führt. Da die Metastasenbildung eines der Hauptprobleme von Krebs ist, bedeutet dies, daß die Inhibition oder Erniedrigung der Überexpression und/oder veränderten Aktivität zu einem wirksamen Mittel bei der Bekämpfung von Krebs wird, wobei insbesondere die Metastasenbildung inhibiert wird.

Mögliche Inhibitoren sind beispielsweise in WO 97113771 oder in Mohammadi et al. (1997) beschrieben. Weder Mohammadi et al. noch WO 97/13771 beschreiben jedoch Inhibitoren von FGFR-4 oder mutiertern FGFR-4 (siehe oben).

Vorzugsweise wird gemäß der Erfindung in eine Überexpression und/oder veränderte Aktivität der Rezeptortyrosinkinase eingegriffen, die durch eine Mutation des FGFR-4 ausgelöst wird. Bei dieser Mutation kann es sich um eine oder mehrere Punktmutationen handeln. Insbesondere tritt die Mutation/die Mutationen in der Transmembrandomäne von FGFR-4 auf, wobei insbesondere eine hydrophobe gegen eine hydrophile Aminosäure ausgetauscht wird.

Es ist bereits bekannt, daß Punktmutationen, die zu einem Austausch hydrophober zu hydrophiler Aminosäuren in FGFR-3 geführt haben, mit bestimmten Krankheiten assoziiert sind. So wurde z.B. eine veränderte Aktivität des Fibroblasten-Wachstumsfaktorrezeptor 3 durch eine Punktmutation in der Transmembrandomäne bei der Achondroplasia gefunden. Die Achondroplasia, bei der es sich um die am häufigsten auftretende genetische Form des Zwergenwuchses handelt, ist eine autosomale dominante Störung, die im wesentlichen auf einem Defekt im Reifungsprozeß bestimmter Knochen beruht. Es konnte nachgewiesen werden, daß die Achondroplasia durch eine Gly- zu Arg-Substitution in der Transmembrandomäne des FGFR-3 ausgelöst wird. Es konnte ferner nachgewiesen werden, daß die Arg-Mutation im FGFR-3 die Kinasefunktion des dimeren Rezeptors aktiviert. Die Arg-Punktmutation führt auch zu einer Liganden-unabhängigen Stimulation der Tyrosinkinaseaktivität von FGFR-3 selbst und zu stark vergrößerten veränderten Niveaus des Phosphotyrosins am Rezeptor. Diese Ergebnisse legen nahe, daß die molekulare Basis der Achondroplasia eine ungeregelte Signalübertragung durch FGFR-3 ist.

Eine weitere Mutation in der Transmembrandomäne von FGFR-3 ist ein Austausch von Alanin gegen Glutamin. Dieser Aminosäureaustausch führt zu einer anderen Krankheit, nämlich zu Crouzon mit Acanthosis nigricans.

Gemäß der vorliegenden Erfindung wurde festgestellt, das Mutationen in FGFR-4, insbesondere Punktmutationen in der Transmembrandomäne, die zu einem Austausch einer hydrophoben gegen eine hydrophile Aminosäure führen, an der Auslösung und schlechten Prognose von Krebs beteiligt sind, weshalb eine Inhibition von Rezeptortyrosinkinasen oder die Verwendung Kinase-inaktiver Rezeptoren für die Behandlung und/oder Prophylaxe von Krebs geeignet sind, wobei die Rezeptortyrosinkinasen aufgrund einer Mutation überexpremieren oder verändert aktiv sind.

Insbesondere konnte für die Punktmutation an Position 388, die zu einem Austausch von Glycin gegen Arginin führt, nachgewiesen werden, daß durch sie die Rezeptortyrosinkinasen verändert aktiv werden und es dadurch homo- wie heterozygot zu einer Signalübertragung ohne Ligandenstimulation kommt, wodurch wiederum ein unkontrolliertes Wachstum von Zellen ausgelöst werden kann. Dieses unkontrollierte Wachstum führt im ungünstigsten Fall zu Krebs: Die Transmembrandomäne weist dann die Sequenz (ID Nr. 1) auf:
RYTDI ILYASGS LALAVLL LLARLY,
während die nicht mutierte Domäne die folgende Sequenz (ID Nr. 2) aufweist:
RYTDI ILYASGS LALAVLL LLAGLY.

Es wird angenommen, daß - ohne an eine Theorie gebunden zu sein - die Aktivierung der Rezeptortyrosinkinase, die eine der oben genannten Punktmutationen und insbesondere die Punktmutation an Position 388 trägt, die zu einem Austausch von Glycin gegen Arginin führt, auf einer Stabilisierung des Rezeptors in einer dimeren Konformation beruht, die aufgrund von Wechselwirkungen zustande kommt, durch die Veränderungen der Transmembrandomäne möglich gemacht wurden. Die verstärkte Bildung eines Liganden unabhängigen Dimers führt zu einer erhöhten Rezeptortyrosinkinaseaktivität und zellulärer Transformation. Andere Möglichkeiten der Einwirkung der Punktmutationen auf die Krebsauslösung können z.B. darin begründet liegen, daß die Mutation sich auf die Signalübertragung von FGFR-4 auswirkt, indem die Rezeptorwanderung durch die Membran verhindert wird, die Rezeptor-Dimerisierung mit sich selbst oder mit anderen FGFRs gestört wird, oder indem die Tyrosinkinaseaktivität des Rezeptors beeinflußt wird.

Gemäß der vorliegenden Erfindung konnte gezeigt werden, daß 56% von Patienten mit Brusttumoren aus St. Petersburg (Untersuchung von Biopsien) die Mutation an Position 388, die mit einem Austausch von Glycin gegen Arginin verbunden ist, trugen. Davon waren 45% heterozygot und 11 % homozygot. Dieser signifikant hohe Anteil legt eine Verbindung zwischen der Punktmutation an Position 388 und der Entstehung von Brustkrebs nahe.

In einer weiteren Untersuchung, in der deutsche Patienten mit Brusttumoren untersucht wurden, zeigten nur 43% der Patienten die Punktmutationen an Position 388. Aus der Untersuchung mit Normaigeweben von Krebspatienten und DNA aus Gewebe gesunder Individuen läßt sich ableiten, daß die Mutation eine Keimbahnmutation ist.

Weiterhin wurde auch genomische DNA und cDNA von Zellinien untersucht, um den Anteil der Punktmutation an Position 388 zu bestimmen. Die untersuchten Zellinien stammten von Brusttumoren, normalen Brustepithelial-Zellinien als Vergleich, dem Squamous-Zellcarcinom, Glioblastomen, Neuroblastomen und Gebärmutterkrebs. Bei allen Zellinien, außer bei den normalen Brustepithelial-Zellinien, konnte ein signifikanter Prozentsatz der Punktmutation an Position 388 im FGFR-4-Molekül gefunden werden. Daher ist die oben erwähnte Verwendung von Inhibitoren bzw. Kinase-inaktiven Rezeptoren besonders für die Behandlung von Carcinomen geeignet. Hier bietet sich insbesondere die Behandlung von Neuroblastomen, Gebärmutterkrebs, Pankreaskrebs, jedoch auch anderer Krebsarten, an.

Besonders bevorzugt ist die Verwendung von Inhibitoren, die einen mutierten FGFR-4 hemmen, insbesondere mit der Mutation Gly → Arg an Position 388 in der Transmembrandomäne.

Ferner betrifft die vorliegende Erfindung einen mutierten FGFR-4, der zu einer Überexpression und/oder veränderten Aktivität des Rezeptors in Zellen führt. Vorzugsweise ist dieser mutierte FGFR-4 dadurch gekennzeichnet, daß eine hydrophobe Aminosäure im Wildtyp-Rezeptor gegen eine hydrophile Aminosäure im mutierten Rezeptor ausgetauscht wurde. Besonders bevorzugt ist eine Mutation, die eine Punktmutation ist und in der Transmembrandomäne auftritt. Noch bevorzugter tritt die Punktmutation an Position 388 auf, wobei vorzugsweise ein Glycin durch Arginin ersetzt wird.

Bis jetzt war man in der Fachwelt davon ausgegangen, daß lediglich ein FGFR-4 vorkommt, der nicht mutiert ist. Mutierte FGFR-4 waren nicht bekannt. Es war daher überraschend, daß gemäß der vorliegenden Erfindung festgestellt werden konnte, daß ein mutierter FGFR-4 existiert. Insbesondere konnte gemäß der vorliegenden Erfindung ein Zusammenhang zwischen den Mutationen, insbesondere der Punktmutation an Position 388, und der Entstehung von Krebs nachgewiesen werden. Weiterhin ist die Keimbahnmutation bei gesunden Personen in Zusammenhang mit der genetischen Prädisposition für das Auftreten u.a. von Arteriosklerose in Verbindung gebracht worden.

Die Erfindung betrifft ferner ein DNA-Molekül umfassend eine Sequenz, die für einen mutierten FGFR-4 kodiert. Die Erfindung umfaßt außerdem ein RNA-Molekül, umfassend eine RNA-Sequenz, die für einen mutierten FGFR-4 kodiert. Die obigen Sequenzen können für eine Diagnose von Krebs verwendet werden. Dabei können die Sequenzen spezifisch die Mutationen im FGFR-4 erkennen. Das Vorliegen der Mutation im FGFR-4 wird mit einer schlechten Prognose für die Behandlung des Krebses in Beziehung gebracht. Grund hierfür könnte ein aggressiveres Wachstumsverhalten des entsprechenden Tumors sein.

Die Erfindung betrifft außerdem ein Verfahren zur Differentialdiagnose von Brustkrebs, wobei die Nukleinsäure des Patienten mit einer der oben beschriebenen DNAs und/oder RNAs in Kontakt gebracht wird, so daß ein Signal erhalten wird, das die Anwesenheit und/oder Abwesenheit von mutiertern FGFR-4 anzeigt. Schließlich betrifft die vorliegende Erfindung eine pharmazeutische Zusammensetzung, umfassend den Inhibitor oder den Kinase-inaktiven Rezeptor, wie oben beschrieben. Die Erfindung betrifft außerdem ein Screeningverfahren zum Identifizieren von Inhibitoren der Tyrosinkinaseaktivität, wobei der erfindungsgemäße Rezeptor mit potentiellen Inhibitoren in Kontakt gebracht wird und die Tyrosinkinaseaktivität in Abwesenheit und/oder Anwesenheit des Inhibitors ermittelt wird.

Weiterhin kann der Nachweis auf das Vorliegen einer Mutation auch mittels PCR und nachfolgender Restriktionsenzymspaltung geführt werden, wie oben bereits ausführlicher beschrieben.

Schließlich kommen auch weitere molekularbiologische Diagnoseverfahren in Betracht.

Gegenstand der Erfindung ist ferner ein Antikörper, der mit einem mutierten FGFR-4 gemäß der Erfindung spezifisch reagiert. "Spezifisch" im Sinne der Erfindung bedeutet, daß der erfindungsgemäße Antikörper an den mutierten, jedoch nicht an den nicht-mutierten Rezeptor bindet.

Im folgenden wird die Erfindung durch die Figuren und Beispiele im Detail beschrieben.

Dabei stellt
- Fig. 1: SDS-PAGE einer Immunopräzipitation von FGFR-4 dar (der phosphorylierte FGF-Rezeptor 4 ist durch einen Pfeil markiert),
- Fig. 2: ein Polyacrylamidgel des mutierten FGFR-4,
- Fig. 3: eine Sequenzanalyse der Transmembrandomäne von FGFR-4,
- Fig. 4: die Korrelation zwischen der FGFR-4-Mutation G388R und dem Status der Lymphknoten-Metastasierung (n = Anzahl der Patienten; p = P-value) und
- Fig. 5: die Korrelation zwischen der FGFR-4-Mutation G388R und der rezidiv-freien Überlebenszeit (n = Anzahl der Patienten; p = P-value) dar.

### BEISPIELE

**Zellkultur.** Die humanen Zellinien MDA-MB-453, ZR 75-1, K562 und SKBr3 wurden von der ATCC bezogen. Die einzelnen Bezugsquellen sind der Tabelle am Schluß zu entnehmen. MBA-MD-453, K562 und ZR 75-1 wurden in RPMI (Gibco, Eggenstein) mit 10 % fötalem Kälberserum (Sigma, Taufkirchen) kultiviert. SKBR3 wurde in McCoy's 5a (Gibco, Egenstein) mit 15 % fötalem Kälberserum kultiviert. Alle Zellkulturmedien enthielten Penicillin/Streptomycin (Sigma, Taufkirchen). Die Zellen wurden bei 37°C in Wasserdampfgesättigter Atmosphäre und 8 % CO₂ inkubiert.

**Klonierung FGFR-4**^{**388Arg**}**/wt.** Zur Präparation von RNA aus K562 und MDA-MB-453 Zellen wurden 3 • 10⁷ Zellen mit Guanidinium-Isothiocyanat lysiert und durch Ultrazentrifugation in einem CsCl-Gradienten gereinigt. Die cDNA-Synthese erfolgte durch reverse Transkriptase (Boehringer, Mannheim) und jeweils 10 pmol "random Oligonukleotiden" nach Angaben des Herstellers. 0,5 µl wurden in eine nachfolgende PCR-Reaktion eingesetzt.

FGFR-4^{388Arg} und FGFR-4wt wurden durch die PCR-Reaktion amplifiziert. Dazu wurden folgende Primer eingesetzt: sense-GCTCAGAGGGCGGGCGGGGGTGCCGGCCG; anti-sense CCGCTCGAGTGCCTGCACAGCCTTGAGCCTTGC. Für die PCR-Reaktion wurden eingesetzt: 1,5 U/25 µl Expand-Polymerase (Boehringer, Mannheim) und Reaktionspuffer laut Angaben des Herstellers; 200 µM dNTP's; 0,01 % v/v Triton X100; 10% v/v DMSO, je 0,2 pmol sense und α-sense Primer. Die folgenden Reaktionsschritte wurden durchgeführt: 35 Zyklen, 94°C 1 min, 64°C 1 min, 72°C 2,5 min. Zur Klonierung von FGFR-4^{388Arg} wurde MDA-MB-453 cDNA eingesetzt und zur Klonierung von FGFR-4wt K562 cDNA. Die PCR-Produkte wurden in den pcDNA3-Vektor (Invitrogen) kloniert. Dadurch konnte sowohl ein FGFR-4 mit der G388R als auch ein Wildtyp-FGFR-4 für weitere Tests erhalten werden.

**Amplifikation der Transmembrandomäne des FGFR-4.** Folgende Primer wurden verwendet; sense-GACCGCAGCAGCGCCCGAGGCCAG; anti-sense-AGAGGGAAGAGG GAGAGCTTCTG. Für die PCR-Reaktion wurden eingesetzt: 1,5 U/25 µl Taq-Polymerase (Boehringer, Mannheim) und Reaktionspuffer laut Angaben des Herstellers; 200 µM dNTP's; je 0,2 pmol sense und α-sense Primer, 0,5 µl cDNA oder genomische DNA aus Tumor-Biopsien und Zellinien; folgende Reaktionsschritte wurden durchgeführt: 35 Zyklen; 95°C 45 sec., 72°C 45 sec.

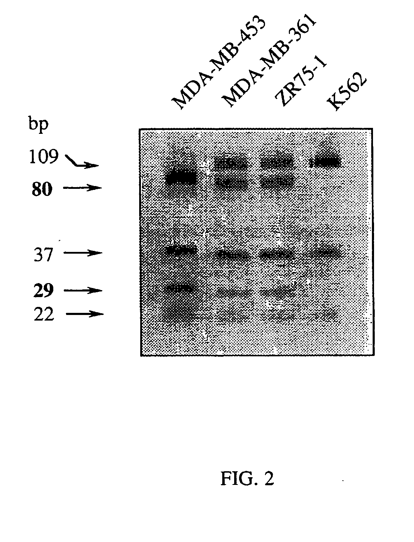

**Analyse durch Restriktionsverdau.** Aus genomischer oder cDNA wurde die Transmembrandomäne des FGFR-4 wie oben beschrieben amplifiziert. Um Biopsien und Zellinien auf die G1217A-Mutation durch einen Restriktionsverdau zu untersuchen, wurden die PCR-Produkte mit 5 U/25 µl BstN1 (NEB, Schwalbach/Taunus), 1 h bei 60°C inkubiert. Die DNA-Fragmente des Restriktionsverdaus wurden durch ein 20%iges Polyacrylamidgel getrennt und mit Ethidiumbromid angefärbt. Die Analyse des Wildtyprezeptors ergibt ein 109, ein 37 und ein 22 Basenpaare großes Fragment (Spur 4). Durch die Mutation G1217A dagegen entsteht eine weitere Restriktionsschnittstelle für BstN1. Der mutierte Rezeptor zeigt weitere 80 und 29 Basenpaare große Fragmente, während das 109 Basenpaare große Fragment verschwindet (Spur 1: homozygot; Spur 2 und 3: heterozygot) (siehe Figur 2).

**Genotypische Analyse von genomischer DNA durch Restriktionsverdau.** Genomische DNA aus den Gewebeproben der primären Tumore wurde durch Standardmethoden isoliert (Current Protocols in Molecular Biology, John Wiley and Sons, Inc., 1995). Um die genomische DNA genotypisch analysieren zu können, wurde die Transmembranregion im FGFR-4 Gen mit folgenden Primern in einer PCR-Reaktion amplifiziert: 5'-GACCGCAGCAGCGCCCGAGGCCAG-3' (bp 1129-1142), 5'-AGAGGGAAGAGGGAGAGCTTCTG-3' bp 1275-1297). Für die PCR-Reaktion wurden Ready-to-Go PCR Beats (Pharmacia, Uppsala, Schweden) verwendet. Folgende PCR-Zyklen wurden benutzt: 3 min bei 95°C, 45 sec bei 94°C, 45 sec bei 72°C und 5 min bei 72°C. Insgesamt wurden 35 Zyklen durchgeführt. Das PCR-Produkt wurde mit 5 U/25 µl BstN1 (NEB, Schwalbach/Taunus) 1 Stunde bei 60°C inkubiert. Die DNA-Fragmente des Restriktionsverdaus wurden durch ein 12%iges Polyacrylamidgel getrennt und mit Ethidiumbromid angefärbt. Das ³⁸⁸Arg-Allel ist durch zwei Fragmente von 80 und 29 bp Grösse charakterisiert, während das ³⁸⁸Gly-Allel durch ein einziges 109 bp großes Fragment angezeigt wird. Jeder genotypische Analyse wurde dreimal wiederholt.

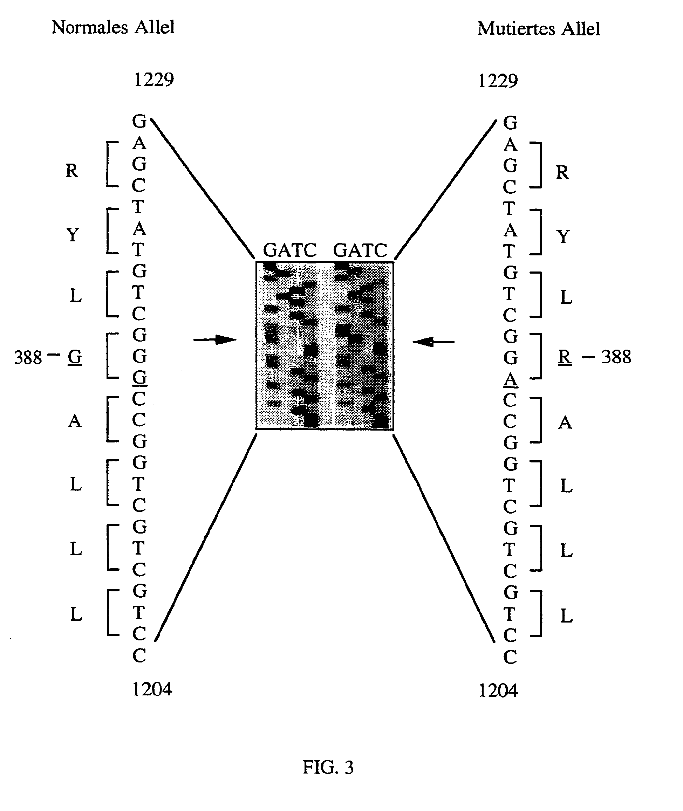

**DNA-Sequenzierung von PCR-Produkten.** Zur Sequenzanalyse der Transmembrandomäne des FGFR-4 wurden die PCR-Produkte in den Bluescript-Vektor kloniert. Dazu wurde eine PCR-Reaktion wie bereits beschrieben durchgeführt. Folgende Primer wurden verwendet: sense-GGGAATTCGACCGCAGCAGCGCCCGAGG; α-sense-GCTCT AGAAGAGGGAAGAGGGAGAG. Die PCR-Produkte der Klonierung von FGFR-4^{Arg388}/wt konnten direkt im Vektor pcDNA3 sequenziert werden. Die DNA-Sequenzierung von Plasmid-DNA wurde nach der Kettenabbruchmethode durchgeführt. Nach dem Annealing des T/-Primer an die Plasmid-DNA erfolgte die Sequenzreaktion mit T/-DNA-Polymerase (Pharmacia, Freiburg). Die Produkte der Sequenzreaktion wurden dann auf einem denaturierenden 5 %igen Polyacrylamidgel (7,5 M Harnstoff; 1 x TBE) getrennt und nach dem Trocknen auf Röntgenfilmen exponiert (siehe Fig.3). Hieraus ergaben sich die DNA-Sequenz des Wildtyps als auch der Mutation.

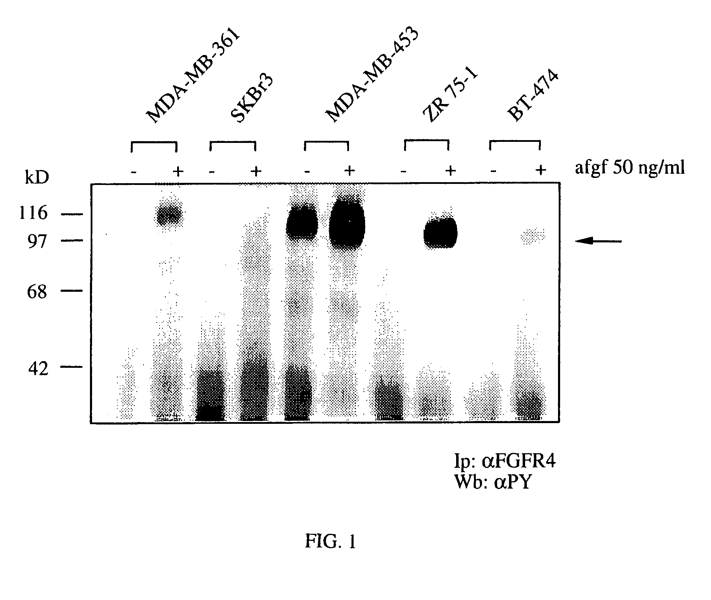

**Immunpräzipitation und Western-Blot-Analyse.** 2,2 • 10⁶ Zellen wurden auf 10 cm Petrischalen ausgesät und über Nacht inkubiert. Dann wurde das Zellmedium durch Medium ohne fötales Rinderserum ersetzt und weitere 24 h inkubiert. Zur Stimulation wurden die Zellen 10 min mit 50 ng aFGF/ml inkubiert, zweimal mit kaltem PBS gewaschen und auf Eis gestellt. Die Zellen wurden mit 300 µl kaltem Lysispuffer (1 % w/w NP-40; 1,25 % w/v Natriumdeoxycholat; 0,1 % w/v SDS, 0,15 M NaCl; 0,01 M Natriumphosphat, pH 7,2; 2 mM EDTA; 10 mM Natriumfluorid; 1 mM PMSF; 20 µg/ml Aprotinin; 1 mM Orthovanadat; 10 mM Natriumpyrophosphat) bei 4°C 15 min inkubiert und das Lysat durch Zentrifugation (13000 upm) bei 4°C geklärt. Zur Proteinwertbestimmung wurde der Mikro-BCA-Protein-Assay (Pierce) nach Angaben des Herstellers verwendet. Zur Immunpräzipitation wurden die Zelllysate auf eine gleiche Proteinmenge eingestellt und dann mit 0,5 µg Anti-FGFR-4 (Santa Cruz) und Protein-A-Sepharose (Pharmacia Freiburg) 18 h bei 4°C auf einem Drehrad inkubiert. Die Immunkomplexe wurden 4 mal mit kaltem HNTG (20 mM HEPES pH 7,5; 150 mM NaCl; 0,1 % Triton X100; 10 % Glycerin; 10 mM Natriumpyrophosphat) gewaschen. Zur Probenvorbereitung wurden die Immunkomplexe mit 50 µl 3 x Laemmli-Puffer versetzt und 5 min bei 99°C inkubiert. Die präzipitierten Proteine wurden auf einem 7,5 %igen SDS-PAGE getrennt (siehe Figur 1).

Für Western-Blots wurden die durch SDS-PAGE getrennten Proteine auf Nitrozellulose transferiert. Unspezifische Proteinbindungsstellen auf der Membran wurden durch Inkubation für 2 h mit TBS-T/0,25 % Gelatine (10 mM Tris/HCl pH 8,0; 0,15 M NaCl; 0,05 % Tween20) bei Raumtemperatur blockiert. Die Inkubation mit primären Antikörpern erfolgte 6 h bei 4°C auf einem Kippschüttler. Unspezifisch gebundene Antikörper wurden dann durch 4 mal Waschen mit TBS-T/0,25 % Gelatine entfernt. Die Bindung sekundärer Antikörper erfolgte 1 h bei Raumtemperatur. Durch einen weiteren Waschschritt wurden die unspezifisch gebundenen sekundären Antikörper entfernt. Immunkomplexe wurden durch den ECL™-Kit (Amersham, Braunschweig) nach Angaben des Herstellers sichtbar gemacht.

**Statistische Methoden.** Statistische Berechungen wurden mit Hilfe der Statistikprogramme MedCalc (MedCalc Software, Belgien) und Epilnfo 6.04b (CDC, Atlanta, Georgia) durchgeführt. Um die Correlationen zwischen den Genotypen in den verschiedenen Patientengruppen und den klinischen Daten zu bestimmen, wurden eine Odds-Ratio, das Konfidenzintervall (Cl) und eine statistische Signifikanz (P-value) berechnet. Wegen der geringen Anzahl von ³⁸⁸Arg-homozygoten Patienten wurde diese Gruppe für die statistischen Berechnungen mit der Gruppe der ³⁸⁸Arg-heterozygoten Patienten vereinigt.

**Nachweis von FGFR-4 in Tumorzellinien.** Tabelle 1 zeigt die Korrelation zwischen der Expression von RTK und Brustkrebs. Es tritt eindeutig gehäuft eine Expression von RTK bei Zellinien aus Brustkrebs auf, während keinerlei Expression bei Zellinien normaler Brustepithelialzellen nachweisbar ist.

**TABELLE 1**

| **Nachweis von FGFR-4 in Brustkrebs-Zellinien** | |
|---|---|
| Brustkrebs-Zellinien | Northern Blot |
| 1 HTB-30(SK-BR-3) | FGFR-4 ++ |
| 2 HTB-122 (BT-549) | - |
| 3 MCF-7 | + |
| 4 BT-483 | +++ |
| 5 T-47D | + |
| 6 ZR-75-1 | +++ |
| 7 MDA-MB-468 | - |
| 8 MDA-MB-453 | ++++ |
| 9 MDA-MB-361 | ++++ |
| 10 MDA-MB-415 | - |
| 11 MDA-MB-231 | - |

| Normale Brustepithelial-Zellinien | |
|---|---|
| 12 HBL-100 | - |
| 13 MCF-10A | - |
| | |

| | |
|---|---|
| Legende: -: keine Expression +: Expression ++: starke Expression +++: sehr starke Expression ++++: extreme Expression | |

Aus Tabelle 2 ist ersichtlich, daß die G388R-Mutation auch bei Zellinien anderer Krebsarten auftritt und mit diesen korrelierbar ist. Bei gesunden Epithelial-Zellinien ist die Mutation nicht nachweisbar.

**TABELLE 2**

| **Mutation FGFR-4 G388R in verschiedenen anderen Tumorzellinien** | | |
|---|---|---|
| Probe | genomische DNA | cDNA |
| Glioblastom | | |
| U-138 | -/- | -/- |
| U-373 | -/- | -/- |
| U-172 | -/- | -/- |
| U-118 | -/* | -/* |
| S F-763 | -/- | -/- |
| U-1240 | */* | */* |
| T-98G | (*)/- | (*)/- |
| U-937 | -/- | -/-. |
| | | |

| Neuroblastom | | |
|---|---|---|
| SK-N-SH | -/* | -/* |
| SH-SY-SY | -/* | -/* |
| | | |

| Gebärmutterkrebs | | |
|---|---|---|
| OAW-42 | -/* | -/* |
| PA-1 | -/- | -/- |
| Caov-3 | -/- | -/- |
| | | |

| Squamous | | |
|---|---|---|
| Hlac-78 | -/- | -/- |
| Hlac-79 | -/- | -/- |
| Scc-4 | -/- | -/- |
| Scc-10a | -/- | -/- |
| Scc-10b | -/- | -/- |
| Scc-17a | -/- | -/- |
| Scc-17b | -/- | -/- |
| Scc-22a | */* | */* |
| Scc-22b | */* | */* |
| HaCat | -/- | -/- |
| FaDu | -/- | -/- |
| | | |

| normale Brustepithelial-Zellinien | | |
|---|---|---|
| HBL-100 | -/- | -/- |
| MCF-10A | -/- | -/- |
| | | |

| | | |
|---|---|---|
| Legende: -/- homozygot unmutiert */- heterozygot mutiert */* homozygot mutiert | | |

**Nachweis der FGFR-4-Mutation G388R in Biopsien.** Tabelle 3 zeigt demnach, daß von 61 untersuchten Patientinnen mit Brustkrebs aus St. Petersburg 56 % die G388R-Mutation trugen, davon 45 % heterozygot und 11 % homozygot. Von den 69 untersuchten Brustkrebspatientinnen aus München trugen 43 % die G388R-Mutation, davon 32 % heterozygot und 11 % homozygot. Der Anteil der Gesamtprozentzahl der Mutation bei Patientinnen aus St. Petersburg bzw. München ist unterschiedlich. Dies legt nahe, daß es sich bei der G388R-Mutation um eine Keimbahnmutation handelt.

**TABELLE 3**

| **Nachweis von FGFR-4-Mutation G388R in Biopsien** | | | | |
|---|---|---|---|---|
| Proben aus Brusttumoren | | | | |
| aus St. Petersburg | | aus München | | |
| Probe | gen. DNA | Probe | gen. DNA | cDNA |
| 19 102 T | */- | 5382 T | */- | */- |
| 20 102 N | | 5609T | */* | */* |
| 21 103 T | */- | 8926 T | */- | */- |
| 22 103 N | */- | 9456 T | */* | */* |
| 23 2 T | */* | 9556 T | */- | */- |
| 24 2 N | */* | 10347 T | -/- | -/- |
| 25 12 T | -/- | 10555 T | -/- | -/- |
| 26 12 N | | 10681 T | */- | */- |
| 27 13 T | -/- | 10781 T | */- | */- |
| 28 13 N | | 10808 T | */* | */* |
| 29 14 T | */- | 11189 T | */- | */- |
| 30 14 N | */- | 11526 T | */- | */- |
| 31 15 T | -/- | 11697 T | */- | |
| 32 15 N | | 11820 T | -/- | -/- |
| 33 17 T | */- | 12015 T | -/- | -/- |
| 34 17 N | */- | 12166 T | */- | */- |
| 35 18 T | -/- | 13932 T | */- | */- |
| 36 18 N | | 16003 T | */- | */- |
| 37 20 T | -/- | 16353 T | -/- | -/- |
| 38 20 N | | 1 N | | |
| 39 21 T | */* | 2 T | */- | */- |
| 40 21 N | */* | 3 N | | |
| 41 22 T | */- | 4 T | -/- | -/- |
| 42 22 N | | 5 N | | |
| 43 23 T | */- | 6 T | -/- | -/- |
| 44 23 N | | 7 N | | |
| 45 31 T | */- | 8 T | -/- | -/- |
| 46 31 N | */- | 9 N | | |
| 47 42 T | -/- | 10 T | */- | */- |
| 48 42 N | | 11 N | | |
| 49 43 T | -/- | 12 T | -/- | -/- |
| 50 43 N | | 13 N | | |
| 51 45 T | -/- | 14 T | */- | */- |
| 52 45 N | | 16 T | */- | */- |
| 53 47 T | */- | 17 N | | -/- |
| 54 47 N | */- | 18 T | | */- |
| 55 48 T | -/- | 19 N | | |
| 56 48 N | | 20 T | | -/- |
| 57 50 T | -/- | 38 T | | -/- |
| 58 50 N | | 3433 T | | -/- |
| 59 53 T | -/- | 3539 T | | |
| 60 53 N | | 3631 T | | */* |
| 61 54 T | */- | 3632 T | | -/- |
| 62 54 N | */- | 3636 T | | -/- |
| 63 55 T | -/- | 3637 T | | */- |
| 64 55 N | | 3638 T | | -/- |
| 65 60 T | */- | 3640 T | | -/- |
| 66 60 N | */- | 991 N | | -/- |
| 67 61 T | */* | 991 T | | -/- |
| 6861 N | */* | 15153 N | | |
| 69 62 T | */- | 15153 T | | -/- |
| 70 62 N | */- | 15856 N | | */* |
| 71 63 T | */- | 15856 T | | */* |
| 72 63 N | */- | 12845 N | | |
| 73 67 T | */- | 12845 T | | -/- |
| 74 67 N | */- | 19044/93 N | | |
| 75 69 T | -/- | 19044/93T | | -/- |
| 76 69 N | | 9426/93 N | | |
| 77 78 T | */- | 9426/93 T | | -/- |
| 78 78 N | */- | 2005 N | | */- |
| 79 79 T | */* | 2005 T | | */- |
| 80 79 N | */* | 14860 N | | |
| 81 82 T | -/- | 14860 T | | -/- |
| 82 82 N | | 4198 T | | -/- |
| 83 83 T | -/- | 5739 T | | */* |
| 84 83 N | | 6060/93 tum | | */* |
| 85 85 T | -/- | 6982/93 tum | | -/- |
| 86 85 N | | 7244/93 tum | | -/- |
| 87 86 T | */- | 8114/93 tum | | -/- |
| 88 86 N | */- | 8335/93 tum | | */- |
| 89 87 T | -/- | 8481/93 tum | | */- |
| 90 87 N | | 8566/93 tum | | -/- |
| 91 89 T | -/- | 8786/93 tum | | */* |
| 92 89 N | | 9145/93 tum | | -/- |
| 93 94 T | -/- | 9354/93 tum | | -/- |
| 94 94 N | | 9796/93 tum | | -/- |
| 95 97 T | */* | 9798/93 tum | | -/- |
| 96 97 N | */- | 10125/93 tum | | -/- |
| 97 98 T | -/- | 10150/93 tum | | */- |
| 98 98 N | | 11218/93 tum | | -/- |
| 99 99 T | */* | 11673/93 tum | | -/- |
| 100 99 N | */* | 13232/93 tum | | -/- |
| 101 100 T | -/- | 13316/93 tum | | */- |
| 102 100 N | | 14724/93 tum | | -/- |
| 103 101 T | */- | 14879/93 tum #1 | | -/- |
| 104 101 N | */- | 14879/93 tum #2 | | -/- |
| 105 102 T (#20) | */- | 15645/93 tum | | -/- |
| 106 102 N (#19) | */* | | | |
| 107 103 T (#22) | */- | | | |
| 108 103 N (#21) | | | | |
| 109 104 T | */- | | | |
| 110 92 T | */* | | | |
| 111 65 T | | | | |
| 112 52 T | */- | | | |
| 113 35 T | */- | | | |
| 114 33 "A" T | */- | | | |
| 115 33 "B" mts. | */- | | | |
| 116 30 T | */- | | | |
| 134 30 N | */- | | | |
| 117 27 T | */* | | | |
| 133 27 N | */* | | | |
| 118 24 T | */- | | | |
| 119 10 T | */- | | | |
| 132 10 T | */- | | | |
| 120 3 T | -/- | | | |
| 121 90 T | -/- | | | |
| 122 90 N | | | | |
| 123 80 T | -/- | | | |
| 124 80 N | | | | |
| 125 81 T | -/- | | | |
| 126 58 T | -/- | | | |
| 127 51 T | */- | | | |
| 128 51 N | | | | |
| 129 44 T | */- | | | |
| 130 44 N | | | | |

**Korrelation zwischen der FGFR-4-G388R-Mutation und dem Nachweis von FGFR-4-Expression.** Aus der folgenden Tabelle 4 geht hervor, daß die G388R-Mutation (genomische DNA und cDNA) nur dann auftritt, wenn eine Expression und/oder verstärkte Expression auftritt. Die Mutation ist weder in den normalen Brustepithelial-Zellinien noch bei den Brustkrebs-Zellinien nachweisbar, bei denen keine RTK-Expression gefunden wurde.

Die Zellinie MDA-MB 453, deren RTK-Expression besonders ausgeprägt ist, zeigt eine homozygote G388R-Mutation.

**TABELLE 4**

| **Korrelation zwischen der FGFR-4-G388R-Mutation und dem Nachweis von FGFR-4-Expression** | | | |
|---|---|---|---|
| Brustkrebs-Zellinien | Northern Blot | Mutation | |
| | FGFR-4 | cDNA | gen. DNA |
| 1 HTB-30 (SK-BR-3) | ++ | +/+ | +/+ |
| 2 HTB-122 (BT-549) | - | -/- | -/- |
| 3 MCF-7 | + | +/- | +/- |
| 4 BT-483 | +++ | +/- | +/- |
| 5 T-47D | + | +/- | +/- |
| 6 ZR-75-1 | +++ | +/- | +/- |
| 7 MDA-MB-468 | - | -/- | -/- |
| 8 MDA-MB-453 | ++++ | +/+ | +/+ |
| 9 MDA-MB-361 | ++++ | +/- | +/- |
| 10 MDA-MB-415 | - | -/- | -/- |
| 11 MDA-MB-231 | - | -/- | -/- |
| | | | |

| Normale Brustepithelial-Zellinien | | | |
|---|---|---|---|
| 12 HBL-100 | - | -/- | -/- |
| 13 MCF-10A | - | -/- | -/- |
| | | | |

| | | | |
|---|---|---|---|
| Legende: -: keine Expression +: Expression ++: starke Expression +++: sehr starke Expression ++++: extreme Expression -/-: keine Mutation +/-: heterozygot mutiert +/+: homozygot mutiert | | | |

**Untersuchung der Korrelation zwischen dem Auftreten der FGFR-4-Mutation G388R und dem Status der Lymphknoten-Metastasierung bzw. der rediviv-freien Überlebenszeit.**

Die Tabelle 5 zeigt die klinischen Parameter aller Patienten, die an der Untersuchung zur Rolle der G388R-Mutation in der Tumorgenese von Brustkrebs teilgenommen haben. Es zeigt sich, daß Patienten mit einer G388R-Mutation eine schlechtere Langzeitprognose haben, als Patienten ohne G388R-Mutation.

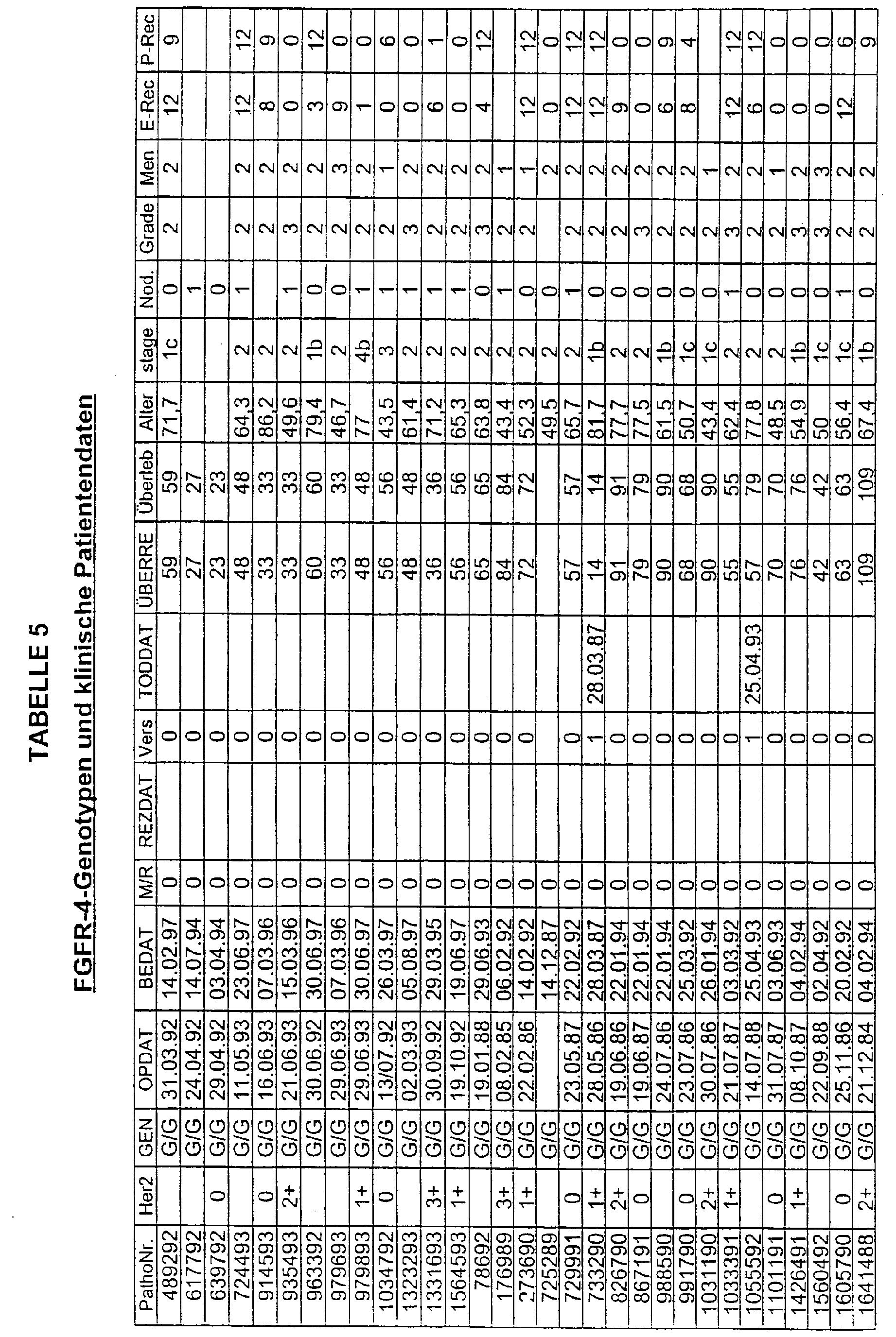

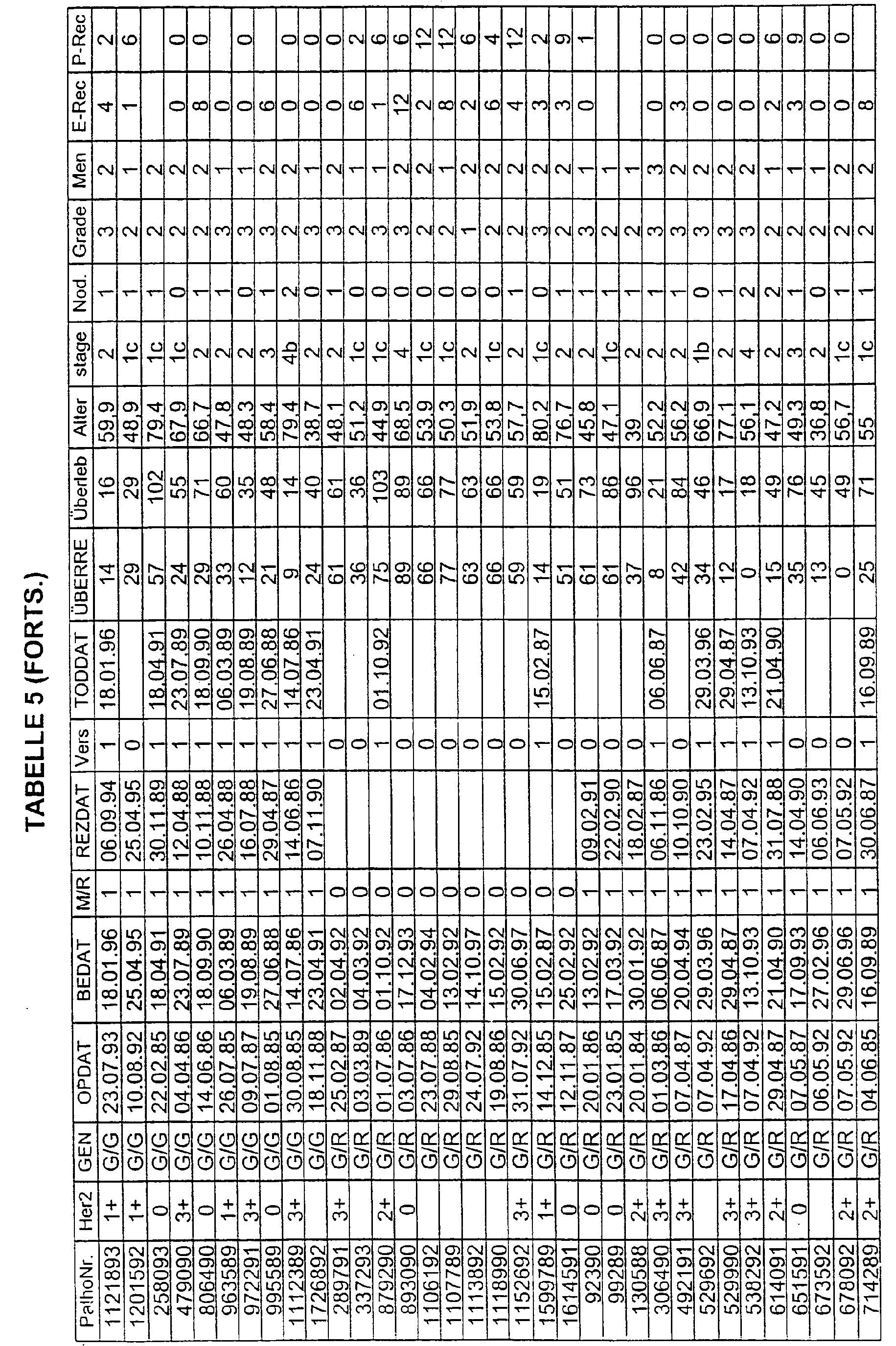

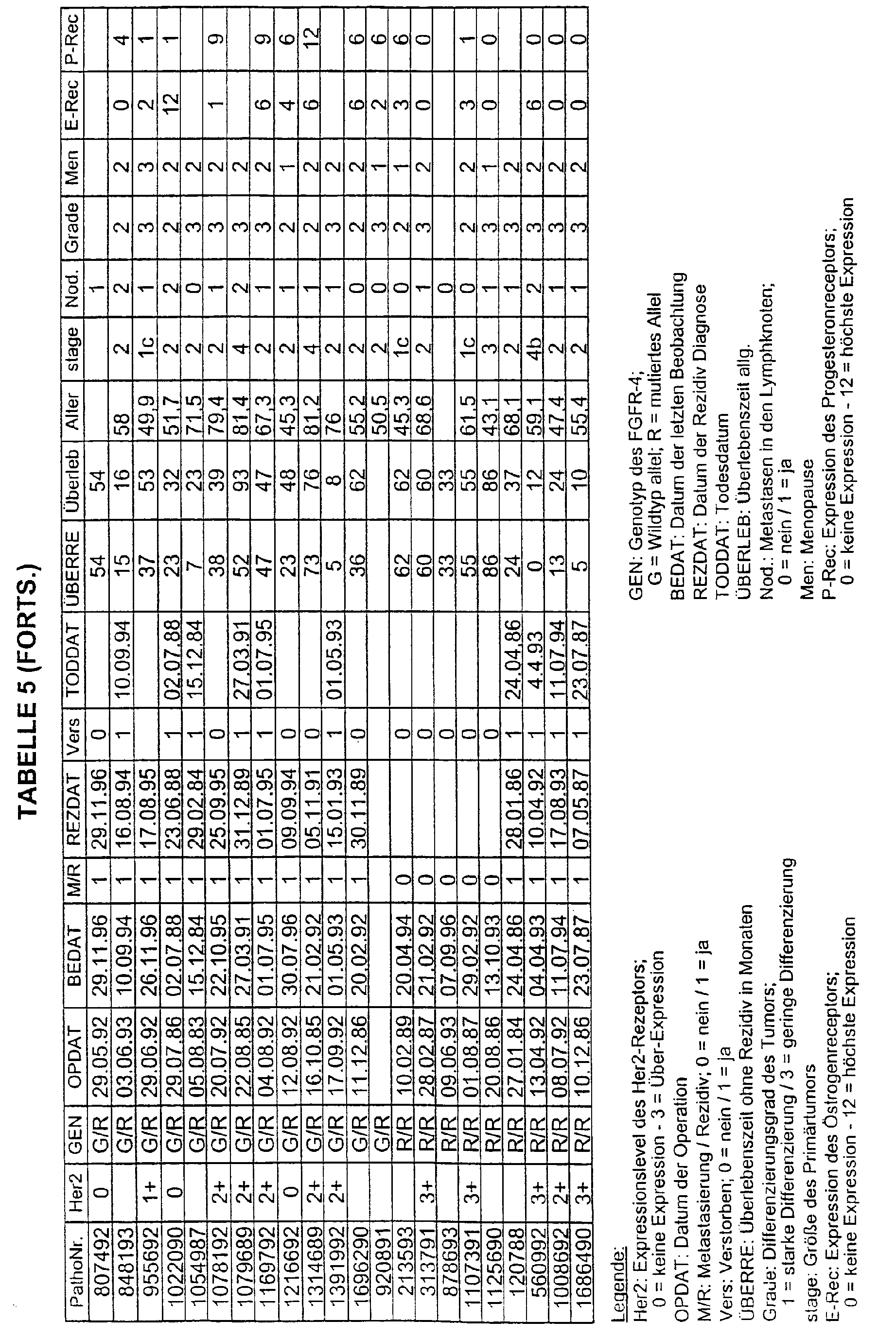

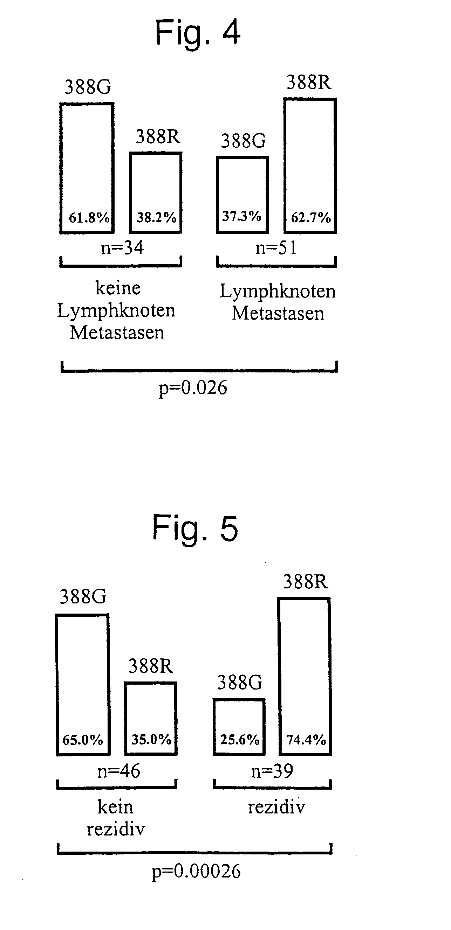

Aus Fig. 4 geht hervor, daß die G388R-Mutation in Patienten, die zur Zeit der ersten Behandlung bereits Metastasen in den Lymphknoten aufwiesen, in einer größeren Anzahl zu finden ist. Von den Patienten mit einer G388R-Mutation hatten 62.7 % Lymphknoten-Metastasen, während von den Patienten ohne G388R-Mutation nur 38.2 % Metastasen in den Lymphknoten zeigten. Da der Status der Lymphknoten-Metastasen ein wichtiger prognostischer Marker zur weiteren Unterscheidung von Tumoren mit einer schlechteren und solche mit einer besseren Prognose darstellt, kann aus diesem Ergebnis geschlossen werden, daß die G388R-Mutation in den untersuchten 85 Patienten zu einer stärkeren Tumorprogression führt.

Aus Fig. 5 ist ersichtlich, daß in der Gruppe der untersuchten Patienten die Rezidiv-freie Überlebenswahrscheinlichkeit sehr viel geringer für diejenigen mit einer G388R-Mutation ist, als für solche Patienten die keine G388R-Mutation aufweisen. Während von den Patienten mit einem Rezidiv 74.4 % den 388R-Genotyp besitzen, haben nur 25.6 % den 388G-Genotyp. Dies zeigt, daß Patienten mit der G388R-Mutation schneller ein Rezidiv bekommen, also nicht erfolgreich therapiert werden konnten.

Zusammenfassend läßt sich feststellen, daß die FGFR-4-Mutation G388R zu einem 2,7fach (OR=2,7; CI: 1,02<OR<7,4) erhöhten Risiko von Metastasenbildung in den Lymphknoten und zu einem 5,44fach (OR=5,44; CI: 1,93<OR<7,4) erhöhten Risiko eines Tumorrezidives führt. Patienten mit einem mutierten FGFR-4-Allel (G388R) scheinen also eine Prädisposition zu einem Tumorrezidiv und damit eine schlechtere Krankheitsprognose zu haben.

### Materialien

| | |
|---|---|
| Acrylamid | Serva, Heidelberg |
| Agar | Difco, Detroit |
| Agarose | BRL, Eggenstein |
| Ampicillin | Boehringer, Mannheim |
| Aprotinin | Sigma, Taufkirchen |
| N,N'-bisacrylamid | Roth, Karlsruhe |
| Cäsiumchlorid | BRL, Eggenstein |
| Desoxynukleotide | Pharmacia, Freiburg |
| Ethidiumbromid | Sigma, Taufkirchen |
| Gelatine | Sigma, Taufkirchen |
| Guanidinium Isothiocyanat | Fluka, Schweiz |
| HEPES | Serva, Heidelberg |
| Natriumfluorid | Sigma, Taufkirchen |
| PMSF | Sigma, Taufkirchen |
| SDS | Roth, Karlsruhe |
| Tris | Riedel de Haen, Seelze |
| Triton X100 | Serva, Heidelberg |
| Tween20 | Sigma, Taufkirchen |

Alle hier nicht aufgeführten Substanzen stammten von den Firmen Sigma (Taufkirchen), Serva (Heidelberg), Riedel Deutsche Patentamt Haen (Seelze) oder Merck (Darmstadt) und wurden in den höchst möglichen Reinheitsgraden eingesetzt.

### Geräte

| | |
|---|---|
| Elektrophorese von DNA | Werkstatt, MPI für Biochemie, Martinsried |
| Elektrophorese von Proteinen | Atto, Japan |
| Kühlzentrifuge Biofuge 17S | Heraeus, Hanau |
| Proteintransfer | Semidry Blot-Apparatur, Werkstatt, MPI für Biochemie, Martinsried |
| Sterilwerkbank | BioGard, The Baker Company, USA |
| Zellkultur | Brutschrank B5060 EK/CO₂, Heraeus, Hanau |
| Zellzahlbestimmung | Coulter Counter, Coulter Electronics, Glasgow |

### Literaturverzeichnis

Basilico C. und Moscatelli D.; The FGF family of growth factors and oncogenes, Advanc. Cancer Res. 59, 115-164 (1992)
Coulier F., De Lapeyriere O. und Birnbaum D.; Complexité de la familie FGF: la preuve par 9. Méd./Sci. 9, 1113-1115 (1993)
Folkmann J. und Klagsbrun M.; Science 235, 442-447 (1987)
Gival D. und Yayon A.; Complexity of FGF receptors: genetic basis for structural diversity and functional specifity, FASEB J. 6: 3362-3369 (1992)
Johnson D.E. und Williams L.T.; Structural and functional diversity in the FGF receptor multigene family, Adv. Cancer Res. 60: 1-41 (1993)
Kimelman D., Abraham J.A., Haaparanta T., Palisi T.M. und Kirschner M.W.; Science 242, 1053-1058 (1988)
Mohammadi M. et al., Science 776, 955-959 (1997)
Slack J.M.W., Darlington G.G., Heath J.K. und Godsave S.F.; Nature 326, 197-200 (1987)
Thomas K.A., Rios-Candelore M., Giminez-Gallego G., DiSalvo J., Bennett C., Rodkey, J. und Fitzpatrick S.; Proc. Natl. Acad. Sci. USA, 82, 6409-6413 (1985)
Thompson J.A., Haudenschild C.C., Anderson K.D., DiPietro J.M., Anderson W.F. und Maciag T. (1989); Proc. Natl. Acad. Sci. USA, 86, 7928-7932 (1989)

| Zellinie | Herkunft | Nr. |
|---|---|---|
| U-138 | ATTC | HTB-16 |
| U-373 | ATTC | HTB-17 |
| U-172 | | |
| U-118 | ATTC | HTB-15 |
| SF-763 | SUGEN | |
| U-1240 | SUGEN | |
| T-98G | SUGEN | |
| U-937 | ATCC | CRL-1593 |
| | | |
| SK-N-SH | ATCC | HTB-11 |
| SH-SY5Y | F.J.Klinz | |
| | | |
| OAW-42 | DKFZ | |
| PA-1 | ATCC | CRL-1572 |
| Caov-3 | ATCC | HTB-75 |
| | | |
| Hlac-78 | Dr. Wustrow | |
| Hlac-79 | Dr.Wustrow | |
| Scc-4 | ATCC | CRL-1624 |
| Scc10a | Dr.Wustrow | |
| Scc10b | Dr.Wustrow | |
| Scc22a | Dr.Wustrow | |
| Scc22b | Dr.Wustrow | |
| Scc17a | Dr.Wustrow | |
| Scc17b | Dr.Wustrow | |
| FaDu | Dr.Wustrow | |
| HaCat | | |
| | | |
| HBL100 | ATCC | HTB-124 |
| MCF10A | ATCC | CRL-10317 |
| | | |
| SKBr-3 | ATCC | HTB-30 |
| BT-549 | ATCC | HTB-122 |
| MCF-7 | ATCC | HTB-22 |
| BT483 | ATCC | HTB-121 |
| T-47-D | ATCC | HTB-133 |
| ZR-75-1 | ATCC | CRL-1500 |
| MDA-MB-468 | ATCC | HTB-132 |
| MDA-MB-453 | ATCC | HTB-131 |
| MDA-MB-361 | ATCC | HTB-27 |
| MDA-MB-415 | ATCC | HTB-128 |
| MDA-MB-231 | ATCC | HTB-26 |
| | | |
| K-562 | ATCC | CCL-243 |

## Patentansprüche

1. Verwendung von mindestens einem Inhibitor von FGFR-4 zur Herstellung eines Medikaments für die Behandlung und/oder Prophylaxe von Rezeptortyrosinkinase (RTK)-Überfunktions-bedingten Beeinträchtigungen, insbesondere von Krebs.

2. Verwendung gemäß Anspruch 1, **dadurch gekennzeichnet, dass** der Inhibitor ein Kinase-inaktiver Rezeptor ist.

3. Verwendung gemäß Anspruch 1 oder Anspruch 2, **dadurch gekennzeichnet, dass** eine Überexpression und/oder veränderte Aktivität von FGFR-4 erniedrigt und/oder inhibiert wird.

4. Verwendung gemäß Anspruch 3, **dadurch gekennzeichnet, dass** die Überexpression und/oder veränderte Aktivität von FGFR-4 durch eine Mutation des FGFR-4 ausgelöst wird.

5. Verwendung gemäß Anspruch 4, **dadurch gekennzeichnet, dass** es sich bei der Mutation um eine oder mehrere Punktmutationen handelt.

6. Verwendung gemäß Anspruch 5, **dadurch gekennzeichnet, dass** die Mutation(en) in der Transmembrandomäne von FGFR-4 auftritt.

7. Verwendung gemäß Anspruch 5 oder Anspruch 6, **dadurch gekennzeichnet, dass** die Mutation(en) zu einem Austausch einer hydrophoben gegen eine hydrophile Aminosäure führt.

8. Verwendung gemäß einem der Ansprüche 5 bis 7, **dadurch gekennzeichnet, dass** die Punktmutation an AS-Position 388 im FGFR-4-Molekül auftritt.

9. Verwendung gemäß einem oder mehreren der vorstehenden Ansprüche 6 bis 8, **dadurch gekennzeichnet, dass** die Punktmutation an AS-Position 388 zu einem Austausch von Glycin gegen Arginin führt (G388R).

10. Verwendung nach einem oder mehreren der Ansprüche 4-9, **dadurch gekennzeichnet, dass** die Mutation(en) Keimbahnmutationen sind.

11. Verwendung nach einem oder mehreren der vorstehenden Ansprüche für die Behandlung von Krebs und/oder auf Hyperproliferation und/oder Invasion zurückzuführende Erkrankungen, insbesondere von Carcinomen, insbesondere durch Inhibition der Metastasenbildung.

12. Verwendung nach einem oder mehreren der vorstehenden Ansprüche für die Behandlung von Brustkrebs.

13. Verwendung nach einem oder mehreren der Ansprüche 1 bis 11 für die Behandlung von Squamous-Zell-Carcinomen.

14. Verwendung nach einem oder mehreren der Ansprüche 1 bis 11 für die Behandlung von Glioblastomen.

15. Verwendung nach einem oder mehreren der Ansprüche 1 bis 11 für die Behandlung von Neuroblastomen.

16. Verwendung nach einem oder mehreren der Ansprüche 1 bis 11 für die Behandlung von Gebärmutterkrebs.

17. Verwendung nach einem der Ansprüche 1 bis 16, **dadurch gekennzeichnet, dass** der Inhibitor einen mutierten FGFR-4 hemmt.

18. Mutierter FGFR-4, der zu einer Überexpression und/oder veränderten Aktivität des Rezeptors in Zellen führt.

19. Mutierter FGFR-4 nach Anspruch 18, **dadurch gekennzeichnet, dass** eine hydrophobe Aminosäure im Wildtyp-Rezeptor gegen eine hydrophile Aminosäure im mutierten Rezeptor ausgetauscht wurde.

20. Mutierter FGFR-4 nach Anspruch 18 oder 19, **dadurch gekennzeichnet, dass** die Mutation eine Punktmutation ist und vorzugsweise in der Transmembrandomäne auftritt.

21. Mutierter FGFR-4 nach Anspruch 18, **dadurch gekennzeichnet, dass** die Punktmutation an Position 388 auftritt, und vorzugsweise ein Glycin durch Arginin ersetzt wird.

22. Polypeptid, umfassend die Sequenz von SEQ ID NO:1.

23. DNA-Molekül, umfassend eine Sequenz, die für einen mutierten FGFR-4 nach einem der Ansprüche 18 bis 21 kodiert.

24. RNA-Molekül, umfassend eine RNA-Sequenz, die für einen FGFR-4 nach einem der Ansprüche 18 bis 21 kodiert.

25. DNA oder RNA-Molekül, umfassend eine Sequenz , die die Aminosäuresequenz von SEQ ID NO: 1 kodiert.

26. Verwendung der Sequenzen gemäß einem der Ansprüche 24 und 25 zur in-vitro Diagnose von Krankheiten, insbesondere von Krebs.

27. Verwendung nach Anspruch 26, **dadurch gekennzeichnet, dass** die Sequenz spezifisch die Punktmutation an Position 388 des FGFR-4 detektieren kann.

28. In-vitro Diagnoseverfahren, insbesondere zur Differentialdiagnose von Krebs, umfassend den Schritt des Nachweisens eines mutierten FGFR-4-Proteins oder eine Nukleinsäure, kodierend hierfür, in einer Patientenprobe.

29. Diagnoseverfahren nach Anspruch 28, **dadurch gekennzeichnet, dass** Nukleinsäure des Patienten mit einer DNA und/oder RNA in Kontakt gebracht wird, so dass ein Signal erhalten wird, das die Anwesenheit und/oder Abwesenheit von mutiertern FGFR-4 anzeigt, und/oder Nukleinsäure des Patienten mit PCR amplifiziert und anschließend mit einem Restriktionsenzym gespalten wird, dessen Erkennungssequenz von der Mutation betroffen ist und/oder Protein des Patienten wird mit einem Antikörper in Kontakt gebracht, der spezifisch ist für das mutierte Protein.

30. In-vitro Verfahren zum Screenen auf das Vorliegen einer genetischen Prädisposition für das Auftreten von Krebs und/oder anderen Erkrankungen umfassend den Schritt des Nachweisens eines mutierten FGFR-4-Proteins oder einer Nukleinsäure, kodierend hierfür, oder einer amplifizierten FGFR-4-Nukieinsäure, in einer Patientenprobe.

31. Pharmazeutische Zusammensetzung, enthaltend einen Kinase-inaktiven FGFR4.

32. In-vitro Screeningverfahren zum Identifizieren von Inhibitoren der Tyrosinkinaseaktivität, wobei der Rezeptor nach einem der Ansprüche 18 bis 21 mit potentiellen Inhibitoren in Kontakt gebracht wird und die Tyrosinkinaseaktivität in Abwesenheit und/oder Anwesenheit des Inhibitors ermittelt wird.

33. Mutierter FGFR-4, vorzugsweise nach einem der Ansprüche 18 bis 21, als Target in der Krebstherapie

34. Antikörper, der mit einem mutierten FGFR-4-Protein nach einem der Ansprüche 18 bis 21 spezifisch reagiert.

## Claims

1. Use of at least one inhibitor of FGFR-4 for manufacture of a drug for the treatment and/or prophylaxis of receptor tyrosine kinase (RTK)-hyperfunction-induced disorders, particularly cancer.

2. Use according to Claim 1, **characterized in that** the inhibitor is a kinase-inactive receptor.

3. Use according to Claim 1 or Claim 2, **characterized in that** an overexpression and/or altered activity of the receptor tyrosine kinase is lowered and/or inhibited.

4. Use according to Claim 3, **characterized in that** the overexpression and/or altered activity of FGFR-4 is triggered by a mutation of FGFR-4.

5. Use according to Claim 4, **characterized in that** the mutation is one or several point mutations.

6. Use according to Claim 5, **characterized in that** the mutation(s) occurs in the transmembrane domain of FGFR-4.

7. Use according to Claim 5 or Claim 6, **characterized in that** the mutation(s) leads to an exchange of a hydrophobic for a hydrophilic amino acid.

8. Use according to one of Claims 5 to 7, **characterized in that** the point mutation(s) occurs at AA position 388 in the FGFR-4-molecule.

9. **Use according to one or several of the foregoing Claims 6 to 8, characterized in that** the point mutation at AA position 388 leads to an exchange of glycine for arginine (G388R).

10. Use according to one or several of the foregoing Claims, **characterized in that** the mutation(s) are germ line mutations.

11. Use according to one or several of the foregoing Claims for the treatment of cancer and/or diseases attributable to hyperproliferation and/or invasion, particularly carcinomas, in particular by inhibition of metastasis formation.

12. Use according to one or several of the foregoing Claims for the treatment of breast cancer.

13. Use according to one or several of Claims 1 to 11 for the treatment of squamous cell carcinomas.

14. Use according to one or several of Claims 1 to 11 for the treatment of glioblastomas.

15. Use according to one or several of Claims 1 to 11 for the treatment of neuroblastomas.

16. Use according to one or several of Claims 1 to 11 for the treatment of uterus cancer.

17. Use according to one of the foregoing Claims 1 to 16, **characterized in that** the inhibitor inhibits a mutated FGFR-4.

18. Mutated FGFR-4, which leads to overexpression and/or altered activity of the receptor in cells.

19. Mutated FGFR-4 according to Claim 18, **characterized in that** a hydrophobic amino acid in the wild type receptor has been exchanged for a hydrophilic amino acid in the mutated receptor.

20. Mutated FGFR-4 according to Claim 18 or 19, **characterized in that** the mutation is a point mutation and preferably occurs in the transmembrane domain.

21. Mutated FGFR-4 according to Claim 18, **characterized in that** the point mutation occurs at position 388, and preferably a glycine is replaced by arginine.

22. Polypeptide, comprising the sequence of SEQ ID NO: 1.

23. DNA molecule, comprising a sequence which codes for a mutated FGFR-4 according to one of Claims 18 to 21.

24. RNA molecule, comprising an RNA sequence which codes for a mutated FGFR-4 according to one of Claims 18 to 21.

25. DNA or RNA molecule, comprising a sequence which codes for the amino acid sequence of SEQ ID NO: 1.

26. Use of the sequences according to one of Claims 24 and 25 in *in vitro* diagnosis of diseases, particularly of cancer.

27. Use according to Claim 26, **characterized in that** the sequence can specifically detect the point mutation at position 388 of FGFR-4.

28. *In vitro* diagnostic method, in particular for the differential diagnosis of cancer, comprising the step of the detection of a mutated FGFR-4 protein or a nucleic acid coding therefor, in a sample of a patient.

29. Diagnostic method according to Claim 28, **characterized in that** nucleic acid of the patient is brought into contact with a DNA and/or RNA, so that a signal is obtained which indicates the presence and/or absence of mutated FGFR-4, and/or nucleic acid of the patient is amplified by PCR and subsequently cleaved with a restriction enzyme whose recognition sequence is affected by the mutation and/or protein of the patient is brought into contact with an antibody which is specific for the mutated protein.

30. ***In vitro* method for screening for the presence of a genetic predisposition for the** occurrence of cancer and/or other diseases comprising the step of the detection of a mutated FGFR-4 protein or a nucleic acid coding therefor, or an amplified FGFR-4 nucleic acid, in sample of a patient.

31. Pharmaceutical composition comprising a kinase-inactive FGFR4.

32. *In vitro* screening method for the identification of inhibitors of tyrosine kinase activity, wherein the receptor according to one of Claims 18 to 21 is brought into contact with potential inhibitors and the tyrosine kinase activity is determined in the presence and/or absence of the inhibitor.

33. Mutated FGFR-4, preferably according to one of Claims 18 to 21, as target in cancer therapy.

34. Antibody, which specifically reacts with a mutated FGFR-4 protein according to one of Claims 18 to 21.

## Revendications

1. Utilisation d'au moins un inhibiteur du FGFR-4 pour la fabrication d'un médicament destiné au traitement et/ou à la prophylaxie de troubles liés à une hyperfonction de récepteurs présentant une activité tyrosine kinase (RTK), notamment le cancer.

2. Utilisation selon la revendication 1, **caractérisée en ce que** l'inhibiteur est un récepteur sans activité kinase.

3. Utilisation selon la revendication 1 ou la revendication 2, **caractérisée en ce qu'**une surexpression et/ou une activité modifiée du FGFR-4 est amoindrie et/ou inhibée.

4. Utilisation selon la revendication 3, **caractérisée en ce que** la surexpression et/ou l'activité modifiée du FGFR-4 est déclenchée par une mutation du FGFR-4.

5. Utilisation selon la revendication 4, **caractérisée en ce qu'**il s'agit, pour la mutation, d'une ou de plusieurs mutations ponctuelles.

6. Utilisation selon la revendication 5, **caractérisée en ce que** la ou les mutations se produisent dans le domaine transmembranaire du FGFR-4.

7. Utilisation selon la revendication 5 ou la revendication 6, **caractérisée en ce que** la ou les mutations conduisent à l'échange d'un acide aminé hydrophobe contre un acide aminé hydrophile.

8. Utilisation selon l'une des revendications 5 à 7, **caractérisée en ce que** la mutation ponctuelle se produit au niveau de l'acide aminé en position 388 dans la molécule de FGFR-4.

9. Utilisation selon l'une ou plusieurs des revendications précédentes 6 à 8, **caractérisée en ce que** la mutation ponctuelle se produit au niveau de l'acide aminé en position 388 pour échanger une glycine contre une arginine (G388R).

10. Utilisation selon l'une ou plusieurs des revendications 4 à 9, **caractérisée en ce que** la ou les mutations sont des mutations germinales.

11. Utilisation selon l'une ou plusieurs des revendications précédentes pour le traitement du cancer et/ou de troubles ayant pour origine une hyperprolifération et/ou une invasion, en particulier pour le traitement de carcinomes, notamment en inhibant la formation de métastases.

12. Utilisation selon l'une ou plusieurs des revendications précédentes pour le traitement du cancer du sein.

13. Utilisation selon l'une ou plusieurs des revendications 1 à 11 pour le traitement de carcinomes à cellules squameuses.

14. Utilisation selon l'une ou plusieurs des revendications 1 à 11 pour le traitement de glioblastomes.

15. Utilisation selon l'une ou plusieurs des revendications 1 à 11 pour le traitement de neuroblastomes.

16. Utilisation selon l'une ou plusieurs des revendications 1 à 11 pour le traitement du cancer de l'utérus.

17. Utilisation selon l'une des revendications 1 à 16, **caractérisée en ce que** l'inhibiteur bloque un FGFR-4 muté.

18. FGFR-4 muté qui conduit à une surexpression et/ou à une activité modifiée du récepteur dans les cellules.

19. FGFR-4 muté selon la revendication 18, **caractérisé en ce qu'**un acide aminé hydrophobe dans le récepteur de type sauvage a été échangé contre un acide aminé hydrophile dans le récepteur muté.

20. FGFR-4 muté selon la revendication 18 ou 19, **caractérisé en ce que** la mutation est une mutation ponctuelle et **en ce qu'**elle se déroule de préférence dans le domaine transmembranaire.

21. FGFR-4 muté selon la revendication 18, **caractérisé en ce que** la mutation ponctuelle se produit en position 388 et remplace de préférence une glycine par une arginine.

22. Polypeptide comprenant la séquence SEQ ID NO : 1.

23. Molécule d'ADN comprenant une séquence codant un FGFR-4 muté selon l'une des revendications 18 à 21.

24. Molécule d'ARN comprenant une séquence d'ARN codant un FGFR-4 selon l'une des revendications 18 à 21.

25. Molécule d'ADN ou d'ARN comprenant une séquence codant la séquence d'acides aminés de la SEQ ID NO : 1.

26. Utilisation des séquences selon l'une des revendications 24 et 25 pour le diagnostic *in vitro* de maladies, notamment du cancer.

27. Utilisation selon la revendication 26, **caractérisée en ce que** la séquence peut détecter spécifiquement la mutation ponctuelle en position 388 du FGFR-4.

28. Procédé de diagnostic *in vitro,* notamment pour le diagnostic différentiel du cancer, comprenant l'étape consistant à mettre en évidence une protéine FGFR-4 mutée ou bien un acide nucléique codant pour celle-ci, dans un échantillon prélevé sur un patient.

29. Procédé de diagnostic selon la revendication 28, **caractérisé en ce que** l'acide nucléique du patient est mis en contact avec un ADN et/ou un ARN, de manière à obtenir un signal qui indique la présence et/ou l'absence du FGFR-4 muté, et/ou l'acide nucléique du patient est amplifié par PCR et ensuite coupé avec une enzyme de restriction dont la séquence d'identification est concernée par la mutation et/ou la protéine du patient est mise en contact avec un anticorps qui est spécifique à la protéine mutée.

30. Procédé *in vitro* pour dépister la présence d'une prédisposition génétique à l'apparition du cancer et/ou d'autres troubles comprenant l'étape consistant à mettre en évidence une protéine FGFR-4 mutée ou un acide nucléique codant pour celle-ci ou bien un acide nucléique de FGFR-4 amplifié dans un échantillon prélevé sur un patient.

31. Composition pharmaceutique contenant un FGFR-4 sans activité kinase.

32. Procédé de dépistage *in vitro* pour identifier des inhibiteurs de l'activité tyrosine kinase, le récepteur selon l'une des revendications 18 à 21 étant mis en contact avec des inhibiteurs potentiels et l'activité tyrosine kinase étant déterminée en l'absence et/ou en présence de l'inhibiteur.

33. FGFR-4 muté, de préférence selon l'une des revendications 18 à 21, servant de cible en oncothérapie.

34. Anticorps qui réagit spécifiquement avec une protéine FGFR-4 mutée selon l'une des revendications 18 à 21.
